(19) Europäisches Patentamt
European Patent Office
Office européen des brevets

(11) **EP 3 549 134 B1**

(12) **EUROPEAN PATENT SPECIFICATION**

(45) Date of publication and mention
of the grant of the patent:
**26.11.2025 Bulletin 2025/48**

(21) Application number: **17816518.9**

(22) Date of filing: **27.11.2017**

(51) International Patent Classification (IPC):
**G16H 20/60** (2018.01)    **G16H 50/30** (2018.01)
**G16H 20/30** (2018.01)    **G16H 50/50** (2018.01)
**A61B 5/00** (2006.01)

(52) Cooperative Patent Classification (CPC):
**G16H 20/30; A61B 5/4866; A61B 5/4875;**
**G16H 20/60; G16H 50/30; G16H 50/50;** A61B 5/01;
A61B 5/024; A61B 5/029; A61B 5/0833;
A61B 5/1072; A61B 5/14532; A61B 5/14546;
A61B 5/208; A61B 5/4266;    (Cont.)

(86) International application number:
**PCT/EP2017/080466**

(87) International publication number:
**WO 2018/099842 (07.06.2018 Gazette 2018/23)**

(54) **DEVICES AND METHODS OF OPTIMAL PERSONALIZED HYDRATION FOR SPORTS**

VORRICHTUNGEN UND VERFAHREN ZUR OPTIMALEN PERSONALISIERTEN
WASSERVERSORGUNG FÜR SPORTARTEN

DISPOSITIFS ET PROCÉDÉS D'HYDRATATION PERSONNALISÉE OPTIMALE POUR LE SPORT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **02.12.2016 US 201662429385 P**

(43) Date of publication of application:
**09.10.2019 Bulletin 2019/41**

(73) Proprietor: **Société des Produits Nestlé S.A.**
**1800 Vevey (CH)**

(72) Inventor: **MARCHAL, Eric**
**88800 Vittel (FR)**

(74) Representative: **Rosolen-Delarue, Katell**
**Société des Produits Nestlé S.A.**
**Avenue Nestlé 55**
**1800 Vevey (CH)**

(56) References cited:
WO-A1-2013/123418    US-A- 6 138 079
US-A1- 2009 157 327

(Cont. next page)

(52) Cooperative Patent Classification (CPC): (Cont.)
A61B 2503/10; A61B 2505/09; A61B 2560/0242

(52) Cooperative Patent Classification (CPC): (Cont.)
A61B 2503/10; A61B 2505/09; A61B 2560/0242

**Description**

BACKGROUND

The present disclosure generally relates to devices and methods for analyzing hydration in an individual. More specifically, the present disclosure relates to an application that can determine an optimal personalized hydration plan for an individual participating in a sport and/or improve athletic performance by the individual.
The combination of exercise and exposure to sunlight and heat can result in dehydration and heat-related conditions such as heat syncope, heat exhaustion, dehydration syndrome, hyponatremia and heat stroke (Casa DJ et al, J Athl Train. 2015 Sep;50(9):986-1000; Coris EE et al, Sports Med. 2004;34(1):9-16). Individuals typically drink when they are thirsty and not to prevent dehydration. However, by the time an individual is thirsty, they are already in the initial stages of dehydration. Even a mild hypohydration by 1% body weight loss can decrease performance in the heat (Bardis CN et all, Medicine and Science in Sports and Exercise Volume 45, Issue 9, September 2013, Pages 1782-1789).
US6138079A discloses a device for the determination of fluid loss by an athlete during training based on physical data. It calculates the amount of fluid being lost by sweating and respiration. The calculation does not take into account water loss from from urine and insensible water loss, as well as endogenic water formed during training.

SUMMARY OF THE INVENTION

The invention is defined by the appended claims.

SUMMARY OF THE DISCLOSURE

The present invention discloses a system as defined by claims 1-15. The examples, aspects and method embodiments, if any, disclosed in the following description that do not fall within the scope of the claims are for reference only, and are to be interpreted as examples useful for understanding various embodiments of the invention.
The present disclosure relates to an application that can confirm if typical water intake by an individual participating in a sport is sufficient to maintain hydration and/or can determine an optimal personalized hydration plan to maintain or improve hydration for an individual participating in a sport. More specifically, the application can estimate water losses during an effort, such as a training session or match, by considering one or more characteristics of the individual, such as weight, height, gender, heart rate at rest, maximal heart rate, $VO_2$ max, effort intensity, and type of clothes worn, and/or one or more characteristics of the training session or match, such as the specific sport, the time duration, and the location of the training session or match (e.g., ambient temperature, moisture, and wind speed). Improved hydration can achieve a corresponding improvement in athletic performance.
Accordingly, in a general embodiment not forming part of the claimed invention, the present disclosure provides a method of decreasing or preventing dehydration in an individual from an athletic training session or match. The method comprises: accepting user input into an application provided by a device comprising a processor, the user input comprising information selected from the group consisting of a characteristic of the individual, a characteristic of the training session or match, and a combination thereof; analyzing the information provided by the user input to determine a water loss by the individual in the athletic training session or match, and the analyzing is performed by the device providing the application; and displaying on the device a personalized hydration plan that comprises at least one instruction selected from the group consisting of an amount of water intake and a frequency of water intake, and the at least one instruction is based at least partially on the water loss determined from the information.
In an embodiment, the characteristic of the individual is selected from the group consisting of weight, height, gender, heart rate at rest, maximal heart rate, $VO_2$ max, effort intensity for the training session or match, a type of clothes worn during the training session or match, and combinations thereof. In an embodiment, the characteristic of the training session or match is selected from the group consisting of a specific sport, a time duration, a position played by the individual, a location, an ambient temperature at the location, a humidity at the location, a wind speed at the location, and combinations thereof.
The analyzing of the information to determine the water loss comprises determining water loss in sweat by the individual in the athletic training session or match, and the at least one instruction is based at least partially on the water loss in sweat by the individual. The information entered by the user input can comprise data comprising a time duration of the training session or match, weight of the individual, height of the individual, age of the individual, gender of the individual, ambient temperature at the location of the training session or match, and optionally wind speed at the location of the training session or match; and the determining of the water loss in sweat by the individual can be based at least partially on the data.
The analyzing of the information to determine the water loss by the individual comprises determining water loss in urine by the individual in the athletic training session or match, and the at least one instruction is based at least partially on the water loss in urine by the individual. The information entered by the user input can comprise data comprising a time duration of the training session or match, heart rate at rest of the individual, age of the individual, and optionally $VO_2$ max of the individual; and the determining of the water loss in urine by the individual can be based at least partially on the data.

The analyzing of the information to determine the water loss by the individual comprises determining respiratory water loss by the individual in the training session or match, and the at least one instruction is based at least partially on the respiratory water loss by the individual. The information entered by the user input can comprise data comprising a time duration of the training session or match, weight of the individual, height of the individual, age of the individual, gender of the individual, ambient temperature and humidity at the location of the training session or match, and optionally wind speed at the location of the training session or match; and the determining of the respiratory water loss by the individual can be based at least partially on the data.

The analyzing of the information to determine the water loss by the individual comprises determining insensible water loss by the individual in the training session or match, and the at least one instruction is based at least partially on the insensible water loss. The information entered by the user input can comprise data comprising a time duration of the training session or match, weight of the individual, height of the individual, and ambient temperature and humidity at the location of the training session or match; and the determining of the insensible water loss can be based at least partially on the data.

In an embodiment, the analyzing of the information to determine the water loss by the individual comprises determining an initial skin temperature of the individual and a final skin temperature of the individual for the training session or match, and the at least one instruction is based at least partially on the initial and final skin temperatures.

The analyzing of the information to determine the water loss by the individual comprises determining an amount of endogenic water formed by the individual in the training session or match. The information entered by the user input can comprise data comprising a time duration of the training session or match, weight of the individual, age of the individual, and gender of the individual; and the determining of the amount of endogenic water formed by the individual can be based at least partially on the data.

In an embodiment, the method further comprises analyzing the information to determine a rate of gastric emptying by the individual, and the at least one instruction comprises a frequency of water intake based at least partially on the rate of gastric emptying by the individual.

In an embodiment, the method further comprises analyzing the information to determine a loss in the training session or match of at least one mineral selected from the group consisting of sodium, calcium and magnesium, and the at least one instruction further comprises an intake amount of the at least one mineral for the individual.

In an embodiment, the method further comprises analyzing the information to determine a glucose loss in the training session or match, and the at least one instruction further comprises an amount of glucose intake for the individual.

In an embodiment, the accepting of the user input comprising the information into the application and the analyzing of the information are completed before the individual participates in the training session or match.

In an embodiment, at least a portion of the amount of water intake is identified for consumption before the training session or match.

In an embodiment, at least a portion of the amount of water intake is identified for consumption during the training session or match.

In an embodiment, at least a portion of the amount of water intake is identified for consumption after the training session or match.

In an embodiment, the water loss in the training session or match is determined by adding a water loss from sweat in the training session or match to water loss from urine in the training session or match and respiratory water loss in the training session or match and subtracting endogenic water in the training session or match.

In an embodiment, the personalized hydration plan improves athletic performance in the individual during the training session or match.

In an embodiment, the characteristic of the training session or match comprises a specific sport such that the analyzing of the information for a first sport is performed using at least one variable or equation different than the analyzing of the information for a second sport different than the first sport even if the information is otherwise identical.

In another embodiment provided by the present disclosure, a method of decreasing or preventing dehydration in an individual during an athletic training session or match comprises accepting user input into an application provided by a device comprising a processor, the user input comprising a usual amount of water intake by the individual in the training session or match and further comprising information selected from the group consisting of a characteristic of the individual, a characteristic of the training session or match, and a combination thereof.

The method comprises analyzing the user input to determine a water loss by the individual in the athletic training session or match, and the analyzing is performed by the device providing the application.

The method comprises displaying an output on the device, the application is configured to display a first output that is a confirmation that the usual amount of water intake by the individual in the training session or match is sufficient to prevent dehydration in the training session or match, the application is configured to display a second output comprising a personalized hydration plan that comprises at least one instruction selected from the group consisting of an amount of water intake and a frequency of water intake, and the at least one instruction is based at least partially on the water loss determined from the information, wherein the output displayed by the application is the first output when the water loss from the usual amount of water intake by the individual in the training session or match is less than a threshold, and the output displayed by the application is the second output when the water loss from the usual amount of water intake by the individual in the training session or match is more than the threshold.

In an embodiment, the characteristic of the training session or match comprises a specific sport such that the personalized hydration plan for a first sport is different than the personalized hydration plan for a second sport different than the first sport even if the information is otherwise identical.

In another embodiment provided by the present disclosure, said embodiment not forming part of the claimed invention, a method of decreasing or preventing dehydration in an individual during an athletic training session or match comprises entering user input into an application provided by a device comprising a processor, the user input comprising information selected from the group consisting of a characteristic of the individual, a characteristic of the training session or match, and a combination thereof. The method comprises administering water to the individual according to a personalized hydration plan displayed in the application by the device, the personalized hydration plan comprises at least one instruction selected from the group consisting of an amount of water intake and a frequency of water intake, and the at least one instruction is based at least partially on a water loss by the individual in the athletic training session or match which is based at least partially on the information in the user input.

An advantage of the present disclosure is to create an optimal personalized hydration plan for an individual participating in a sport.

Another advantage of one or more embodiments provided by the present disclosure is to maintain or improve hydration in an individual participating in a sport.

Still another advantage of one or more embodiments provided by the present disclosure is to guide an individual participating in a sport to use water intake directed to hydration in order to avoid any hypohydration.

Yet another advantage of one or more embodiments provided by the present disclosure is to consider hydration factors not typically addressed for an individual participating in a sport.

Furthermore, another advantage of one or more embodiments provided by the present disclosure is to mitigate or prevent decreased athletic performance that results from dehydration in an individual participating in a sport.

Moreover, another advantage of one or more embodiments provided by the present disclosure is to improve athletic performance by an individual participating in a sport.

An additional advantage of one or more embodiments provided by the present disclosure is to identify a volume and a frequency of water intake for an individual participating in a sport.

Another advantage of one or more embodiments provided by the present disclosure is to predict water loss for an individual participating in a sport.

Still another advantage of one or more embodiments provided by the present disclosure is to prevent or minimize water loss in an individual participating in a sport.

Yet another advantage of one or more embodiments provided by the present disclosure is to consider various types of water loss experienced by an individual participating in a sport.

Furthermore, another advantage of one or more embodiments provided by the present disclosure is an easily navigable interactive tool to create an optimal personalized hydration plan for an individual participating in a sport.

Moreover, another advantage of one or more embodiments provided by the present disclosure is to optimize water intake occurring before, during and/or after participation in a sport.

An additional advantage of one or more embodiments provided by the present disclosure is to use urine loss, respiratory water loss, insensible water loss, and sweat loss to determine water need for an individual participating in a sport.

Another advantage of one or more embodiments provided by the present disclosure is to use the type of clothing worn by an individual participating in a sport to determine water need.

Still another advantage of one or more embodiments provided by the present disclosure is to use endogenic water and water drunk during effort to determine water need for an individual participating in a sport.

Yet another advantage of one or more embodiments provided by the present disclosure is an interactive tool accessible over the internet that requests information from an individual participating in a sport and then uses the information to create a personalized optimal hydration plan for the individual.

Furthermore, another advantage of one or more embodiments provided by the present disclosure is to confirm if the typical hydration by an individual participating in a sport is sufficient to maintain hydration.

Moreover, another advantage of one or more embodiments provided by the present disclosure is to identify the effect on body weight and/or athletic performance of the typical hydration by an individual participating in a sport.

An additional advantage of one or more embodiments provided by the present disclosure is an optimal personalized hydration plan that considers the gastric tolerance of an individual participating in a sport.

Yet another advantage of one or more embodiments provided by the present disclosure is an optimal personalized hydration plan that considers mineral loss and/or glucose loss in an individual participating in a sport and addresses these deficits.

Additional features and advantages are described in, and will be apparent from, the following Detailed Description and the Figures.

BRIEF DESCRIPTION OF DRAWINGS

**FIG. 1** is a graph of experimental findings regarding water losses and water intake by sport.

**FIG. 2** is a graph of experimental findings regarding body weight loss by sport.

**FIG. 3** is a graph of experimental findings regarding individual water losses in a France junior basketball team.

**FIG. 4** is a graph of experimental findings regarding individual water losses in a France professional soccer team.

**FIG. 5** is a graph of experimental findings regarding individual water loss in a France professional cycling team.

**FIG. 6** is a graph of experimental findings regarding individual water loss in a France elite female tennis team.

**FIG. 7** is a graph of experimental findings regarding water losses during training and matches with various conditions for elite handball teams.

**FIG. 8** is a graph of experimental findings regarding water losses in ice hockey players at different ages.

**FIG. 9** is a graph of experimental findings comparing water losses between women and men during tennis training in the same environment.

**FIG. 10** shows determination of the volume of sweat loss during effort ($m_s$) in liters in one or more embodiments of the methods provided by the present disclosure.

**FIG. 11** shows determination of the $VO_2$ max in one or more embodiments of the methods provided by the present disclosure.

**FIG. 12** shows determination of the volume of urine lost during effort ($m_u$) in liters in one or more embodiments of the methods provided by the present disclosure.

**FIG. 13** shows determination of the volume of water lost by breathing during effort ($m_{res}$) in liters in one or more embodiments of the methods provided by the present disclosure.

**FIG. 14** shows determination of the volume of water synthesized by the body ($m_{met}$) in liters in one or more embodiments of the methods provided by the present disclosure.

**FIG. 15** shows determination of the metabolic weight loss ($P_{met}$) in kg in one or more embodiments of the methods provided by the present disclosure.

**FIG. 16** shows determination of the glucose loss ($m_{Glu}$) in g in one or more embodiments of the methods provided by the present disclosure.

**FIG. 17** shows determination of blood sugar after effort ($Gly_f$) in g/L in one or more embodiments of the methods provided by the present disclosure.

**FIG. 18** shows a non-limiting example of determination of an optimal personalized hydration plan.

**FIG. 19** shows determination of water deficit after effort and total body weight loss after effort in the non-limiting example of an optimal personalized hydration plan.

**FIG. 20** shows determination of post-effort hydration in the non-limiting example of an optimal personalized hydration plan.

DETAILED DESCRIPTION

Definitions

Some definitions are provided hereafter. Nevertheless, definitions may be located in the "Embodiments" section below, and the above header "Definitions" does not mean that such disclosures in the "Embodiments" section are not definitions.

All percentages and ratios are by weight unless otherwise specified. As used herein, "about" and "approximately" are understood to refer to numbers in a range of numerals, for example the range of -10% to +10% of the referenced number, preferably within -5% to +5% of the referenced number, more preferably within -1% to +1% of the referenced number, most preferably within -0.1% to +0.1% of the referenced number. A range that is "between" two values includes those two values.

The relative term "preventing dehydration" includes reduction of risk and/or severity of dehydration. The relative term "decreasing dehydration" means that the hydration of the individual utilizing the personalization hydration plan disclosed herein (e.g., consuming the amount of water identified by the plan and/or using the frequency of water consumption identified by the plan) at the completion of the training session or match and/or at a time proximate to the completion of the training session or match (e.g., one hour or thirty minutes after the training session or match) is at least equal to the hydration of the individual when they consume their usual amount of water at their usual frequency of consumption (e.g., at the same relative time with respect to a substantially similar training session or match but without using the personalized hydration plan).

Similarly, the relative term "improving athletic performance" means that the athletic performance (e.g., frequency of beneficial outcomes and/or extent of beneficial outcomes achieved by the individual) during the training session or match by the individual utilizing the personalization hydration plan disclosed herein (e.g., consuming the amount of water identified by the plan and/or using the frequency of water consumption identified by the plan) is better than the athletic performance during the training session or match by the individual when they consume their usual amount of water at their usual frequency of consumption (e.g., at the same relative time with respect to a substantially similar training session or match but without using the personalized hydration plan).

The term "administering" includes both an individual administering the referenced composition to themselves and another individual administering the referenced composition to the individual.

An "effort" is participation in a sporting training session or match. The present disclosure is not limited to a specific sport or a specific training session or match and includes any athletic activity in which a participating individual is at risk of a water deficit such as dehydration.

"Ambient temperature" means the temperature of the immediate surroundings of the training session or match. In some embodiments, the ambient temperature is the actual measured temperature (e.g., the temperature at the location the day before the training session or match or the day of the training session or match). In other embodiments, the ambient temperature is the average temperature of that location in that season, that month, that week, or that day. As used herein, "ambient temperature" does not have its colloquial meaning of the preferred indoor climate-controlled temperature.

The term "database" means the hardware (e.g., a computer) and/or the software (e.g., a computer program such as a computer application) that receives, stores, processes and delivers content that can be accessed, for example, through the internet using a website hosted by the database and/or a web server associated with the database.

The term "automatically" means without user input being necessary. An operation performed "automatically" can comprise one or more actions by the corresponding device, but each of the actions is performed without a requirement of user input.

The terms "provide" and "display" not only include visual representations such as text and/or icons but also include audio representations as well, alone or in combination with visual representations.

The term "module" used herein, and particularly in the figures, is a formula and/or variable determined from tests and may depend on one or more of the sport and the gender and/or weight of the individual participating in the sport. The term "data" used herein, and particularly in the figures, is information input by a user of the application.

As used herein and in the appended claims, the singular form of a word includes the plural, and vice versa, unless the context clearly dictates otherwise. Thus, the references "a," "an" and "the" are generally inclusive of the plurals of the respective terms. For example, reference to "a device" or "a method" includes a plurality of such devices or methods.

The term "and/or" used in the context of "X and/or Y" should be interpreted as "X," or "Y," or "X and Y."

The words "comprise," "comprises," and "comprising" are to be interpreted inclusively rather than exclusively. Likewise, the terms "include," "including" and "or" should all be construed to be inclusive, unless such a construction is clearly prohibited from the context. However, the embodiments provided by the present disclosure may lack any element that is not specifically disclosed herein. Thus, a disclosure of an embodiment defined using the term "comprising" also is a disclosure of embodiments "consisting essentially of" and "consisting of" the disclosed components or steps. Where used herein, the term "example," particularly when followed by a listing of terms, is merely exemplary and illustrative, and should not be deemed to be exclusive or comprehensive.

It is noted that the various aspects, features, examples and embodiments of the devices, systems and methods described in the present disclosure may be compatible and/or combined together, unless otherwise specified.

Embodiments

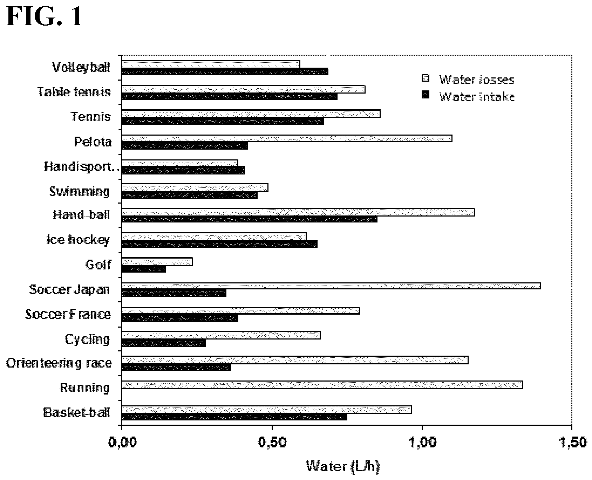

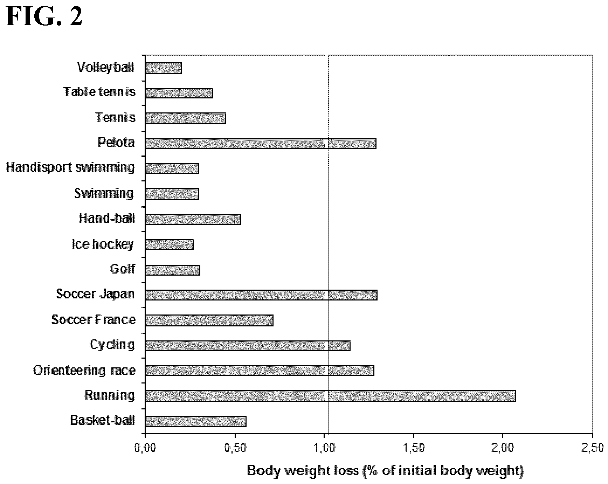

As shown in **FIGS. 1** and **2,** the present inventors found that water losses can vary largely depending on the nature of the sport. Water intakes also depend on the sport due to accessibility of bottles and to awareness of the importance to drink; hydration habits and education are not the same among disciplines.

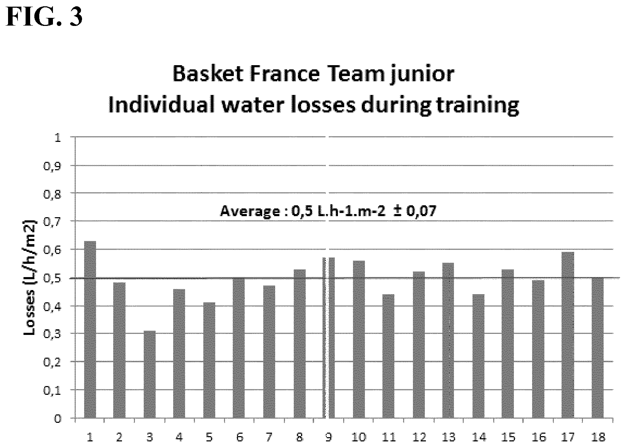

As shown in **FIGS. 3-6,** individual differences between participants can be observed even if the global standard deviation is low. In a first example **(FIG. 3),** the basketball junior France team was studied during a training session, and water losses are expressed in liter per hour per meter square of body surface. The average water loss was 0.5 $L.h^{-1}.m^{-2}$, and the standard variation was 0.07 $L.h^{-1}.m^{-2}$. In this example, 56% of the team had losses within 10% of standard deviation, and 89% had losses within 20% of deviation.

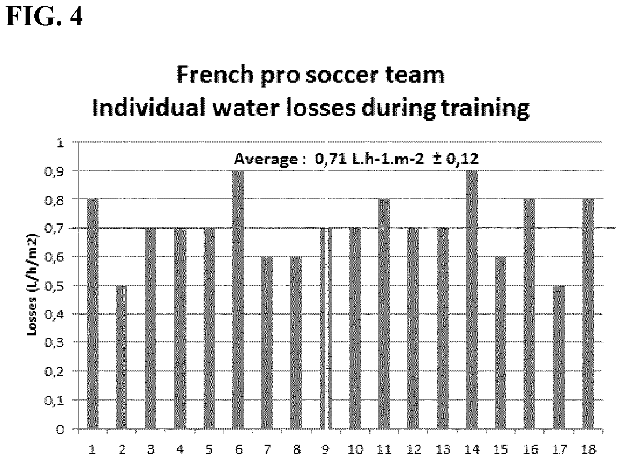

In a second example **(FIG. 4),** a professional soccer team was studied during a training session. The average water loss was 0.71 L.h$^{-1}$.m$^{-2}$, and the standard variation was 0.12 L.h$^{-1}$.m$^{-2}$. In this example, 39% of the team has losses within 10% of standard deviation, and 78% has losses within 20% of deviation.

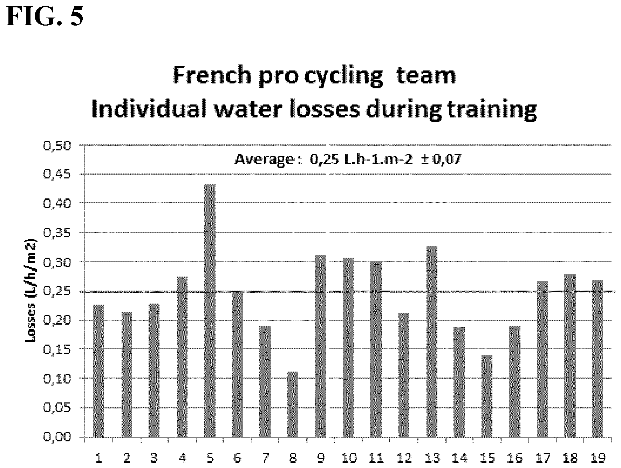

In a third example **(FIG. 5),** a professional cycling team was studied during a training session. The average water loss was 0.25 L.h$^{-1}$.m$^{-2}$, and the standard variation was 0.07 L.h$^{-1}$.m$^{-2}$. In this example, 32% of the team has losses within 10% of standard deviation, and 47% has losses within 20% of deviation. The lowest global losses in cycling can be explained by the fact that heat losses are not only done by sweat but also by convection. Specific equations have been established for cycling, as discussed in greater detail later herein.

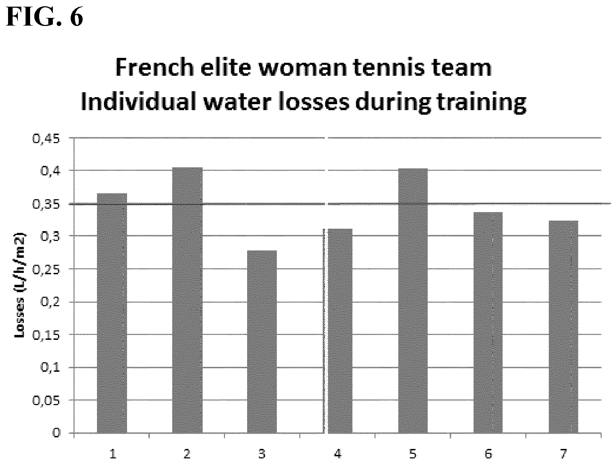

In a fourth example **(FIG. 6),** an elite female tennis team was studied during a training session. The average water loss was 0.35 L.h$^{-1}$.m$^{-2}$, and the standard variation was 0.05 L.h$^{-1}$.m$^{-2}$. 57% of the team has losses within 10% of standard deviation, and 100% has losses within 20% of deviation.

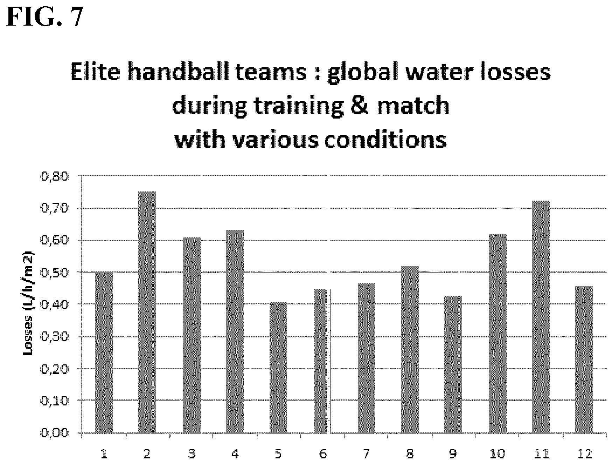

As shown in **FIG. 7,** several elite handball teams were studied during training and matches with various intensities (average 133 beats per minute $\pm$ 8) and conditions of indoor temperature (average 21.3°C $\pm$ 3.7) and humidity (average 49% $\pm$ 15). Global losses for each team session showed huge differences, ranging from 0.41 to 0.75 L.h$^{-1}$.m$^{-2}$. Considering that the indoor atmospheric conditions vary in a rather narrow range, these results show the influence of intensity of effort on water losses during training and matches with various conditions.

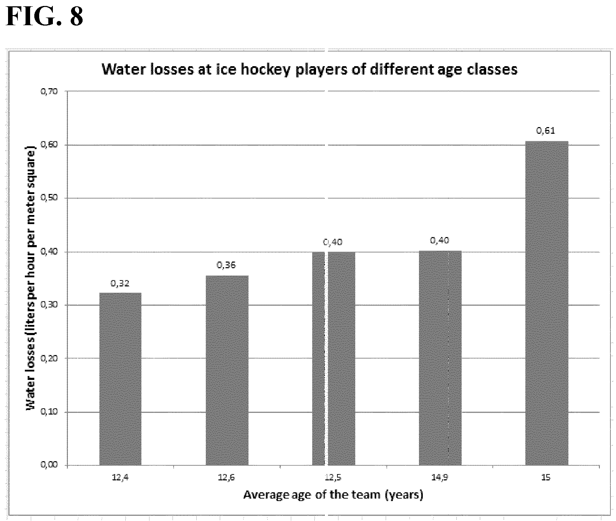

As shown in **FIG. 8,** the age of the participant impacts water loss. For example, children do not sweat like teenagers. Sweat pattern changes during puberty, which explains the observed increase in teams where age class increases from 12 to 15 years old on average.

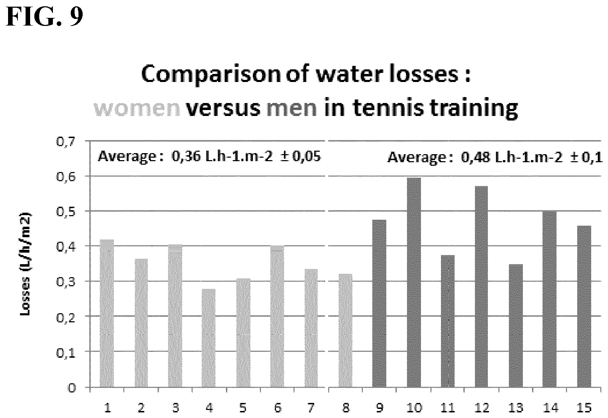

As shown in **FIG. 9,** the gender of the participant impacts water loss. For example, women and men had a tennis training session in the same environment (ambient temperature, intensity), and the women had lower water losses than the men in liter per hour per square meter of body surface: 036 versus 0.48.

In view of these findings, the present inventors developed devices, systems and methods for optimal personalized hydration in an individual participating in a sport. For example, a program executed by a device comprising a processor can provide an application that requests information related to one or more of the sport, the location of the sport, and a characteristic of the individual and provides an optimal personalized hydration plan for the individual. In this regard, some embodiments consider the location of the training session or match because the location can affect water loss due to the temperature and/or the humidity in the air at the location.

The optimal personalized hydration plan can comprise a volume of water intake and/or a frequency of water intake and can comprise water intake times prior to participation in the sport (e.g., times within three hours prior, for example times within two hours prior or times within one hour prior), during participation in the sport, and/or after participation in the sport (e.g., times within three hours after, for example times within two hours after or times within one hour after). The timing of the water intake can depend on characteristics of the specific individual and/or can depend on the specific sport.

The program can estimate water losses during an effort by considering one or more characteristics of the individual, such as weight, height, gender, heart rate at rest, maximal heart rate, VO$_2$ max, effort intensity, and type of clothes worn, and/or one or more characteristics of the training session or match, such as the specific sport, the time duration, and the location of the training session or match (e.g., ambient temperature, moisture, and wind speed), as discussed in greater detailed later herein.

Preferably, the application requests that the user indicate how much water they usually drink during the training session or match. The application can use this value (Ve) to confirm if the typical hydration by the individual is sufficient to maintain hydration to the completion of the training session or match, identify the effect of the typical hydration on body weight and/or athletic performance, and provide the optimal personalized hydration plan that identifies the amount of water intake and/or the frequency thereof to maintain or improve hydration to the completion of the training session or match. Preferably, the application requests that the user identify how many breaks occur during the training session or match so that the amount of water needed can be divided by the number of breaks to determine the amount of water needed per break.

In a preferred embodiment, the program considers water intake, water produced by the body, and all types of water loss to determine global water needs during a specific effort for a specific subject. For example, the program can determine global water needs by adding the amount of water lost from sweat to the amount of water lost by urine and subtracting endogenic water and water intake (need = sweat + urine + respiratory steam - endogenic water - water intake). This determination allows the program to provide customized advice on water need.

Water losses can include urine loss (U), and urine loss can be deduced from several equations and data from literature involving glomerular filtration rate, renal plasma flow, hematocrit, cardiac output, and stroke volume (e.g., Poortmans JR, Sports Medicine, 1:125-153 (1984)). All these factors can be linked to heart rate at rest and VO$_2$ max, and heart rate at rest and VO$_2$ max can be entered by the user or calculated by the program.

$$U = a * 10^{-7} * HRr * (67+20.61 * ((VO_2 \ max * weight/1000)-2.0)) * t$$

with a=2.16 for male and a=2.36 for female, t = length of effort (min), HRr = heart rate at rest (bpm), $VO_2$ max = maximal consumption of oxygen (mL.min$^{-1}$.kg$^{-1}$)

In an embodiment, the data entered by the user to calculate urine loss (U) comprises length of effort (t), heart rate at rest (HRr), age and/or $VO_2$ max (if known). Necessary data not entered by the user can be calculated from equations in the literature.

Water losses can include respiratory loss (R). Respiratory loss due to breathing has been deduced from several literature data (e.g., Kerslake D. McK. The stress of hot environments. Monographs of the Physiological Society n°29, Cambridge, University Press; and Ferrus L. et al. Respiration Physiology, 39:367-381 (1980)) and can be summarized as:

$$R = 0.0023 * M * (P_{sres}-P_a) * Bsa * 60 * t/\lambda$$

with M = metabolic heat (W.m$^2$), $P_{sres}$ = saturated water vapor pressure of respiratory tract (mmHg), $P_a$ = ambient water vapor pressure (mmHg), Bsa = Body surface (m$^2$), $\lambda$ = vaporization latent heat (J.g$^{-1}$)

In an embodiment, the data entered by the user to calculate respiratory loss (R) comprises length of effort (t), weight, height, age, gender, ambient temperature, and optionally wind speed and/or speed of the participant (if relevant; preferably used at least for cycling). Necessary data not entered by the user can be calculated from equations in the literature.

Water losses can include insensible water losses (In), which can occur instead of sweat when activity intensity is low and environmental factors are temperate (ambient temperature, moisture). The insensible water losses can be calculated using equations of the literature (e.g., Tokura et al., Int. J. Biometeor., 22(4):271-278 (1978); Mathias et al., J. Invest. Dermatol., 77:219-220 (1981); and Wilson et al., Br. J. Dermatol., 119:647-652 (1988)).

$$I_n = (6.0 + 1.75 * (P_{sp} - P_a)/7.5) * Bsa * t$$

with $P_{sp}$ = skin water vapor pressure (mmHg)

In an embodiment, the data entered by the user to calculate insensible water losses (In) comprises length of effort (t), weight, height, ambient temperature and humidity. Necessary data not entered by the user can be calculated from equations in the literature.

Water losses can include sweat (S) which occurs when the body becomes unable to evacuate heat only by insensible water losses. Sweat can be calculated by the following equation derived from Shapiro et al (1982) :

$$S = (60 * a * E_{req} * E_{max}^{-b} * t * Bsa) / \lambda f$$

with $E_{req}$ = required evaporative cooling (W.°C$^{-1}$.m$^{-2}$), $E_{max}$ = maximal evaporative capacity (W.mmHg$^{-1}$.m$^{-2}$) and $\lambda f$ = final vaporization heat (J.g$^{-1}$)

Regarding factors a and b in the sweat equation, these two factors were calculated by Shapiro from 15 groups (111 subjects) doing a basic effort lower than 30% $VO_2$ max in different environments for temperature and clothes. In this case, a = 18.7 and b = 0.455. To use this formula for intensity of effort higher than 30% $VO_2$ max, which is the usual case for sport effort, the present inventors gathered raw results from twelve papers and used the results therein to calculate a and b for 3 different cases: (i) short + T-shirt, (ii) tracksuit or (iii) protecting clothes.

In an embodiment, the data entered by the user to calculate sweat (S) comprises length of effort (t), weight, height, gender, ambient temperature, and optionally wind speed and/or speed of the participant (if relevant; preferably used at least for cycling). $E_{req}$, $E_{max}$ and $\lambda f$ are calculated from equations of the literature.

Determination of water losses can include calculating the skin temperature ($T_p$) of the individual. The initial skin temperature ($T_{pi}$) (e.g., at rest or when the training session or match begins) and the final skin temperature ($T_{pf}$) (e.g., when the have been calculated from literature raw data compilation.

$T_{pi}$ is finally linked to dry ambient temperature ($T_a$) with the following equation:

$$T_{pi} = c * (36 + 0.015 * p)$$

with c dependent of the clothes worn

In the same way, $T_{pf}$ has been calculated from raw data of scientific papers representing 221 subjects and is linked to $T_a$:

$T_{pf} = d * T_a + b$ with d and b depending of type of clothes (short + T-shirt versus tracksuit or protection clothes)

Endogenic water ($E_n$) can at least partially offset the water losses discussed above and be calculated from a known formula found in literature. Endogenic water ($E_n$) depends on length of effort (t), weight, age and gender.

Water intake (typically drinking) can be limited by gastric intolerance and emptying. Gastric emptying has been predicted from the raw results obtained by Rehrer et al. (1990) that evaluate the absolute amount of drink remaining in the stomach after a single bolus or after repeated ingestion. Relation between the initial volume ingested ($V_0$) and the volume that leave stomach after time t (Vt) is exponential with time:

$$V_t = V_0 * (0.939)^t$$

The present inventors then extrapolated gastric emptying speed for a bolus intake every i minutes (vi):

$$vi = 0.0615 * (0.97366)^i * V_0$$

This calculation allows the program to calculate the frequency of water intake during the effort. For example, the program can use the gastric emptying speed to determine the rate at which the desired total amount of water should be consumed in separate doses.

In an embodiment, the program determines mineral losses and/or glucose losses in the individual participating in a sport. The hydration plan can include information regarding maintaining a mineral level and/or a glucose level.

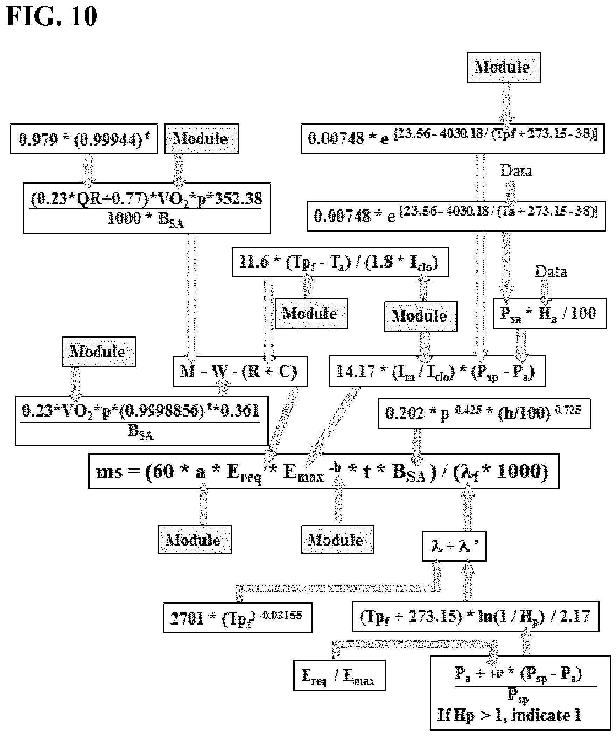

The calculations provided above are general approaches to provide optimal personalized hydration for an individual participating in a sport. Non-limiting examples of specific calculations are shown in **FIGS. 10-16.** In these calculations, the abbreviations at the end of this document are used.

**FIG. 10** generally illustrates an embodiment of calculations for estimating the volume of sweat loss during effort ($m_s$) in liters. In some embodiments, the estimated sweat loss ($m_s$) can be adjusted based on other factors, for example whether the effort was a training session or a match and/or the intensity of the effort.

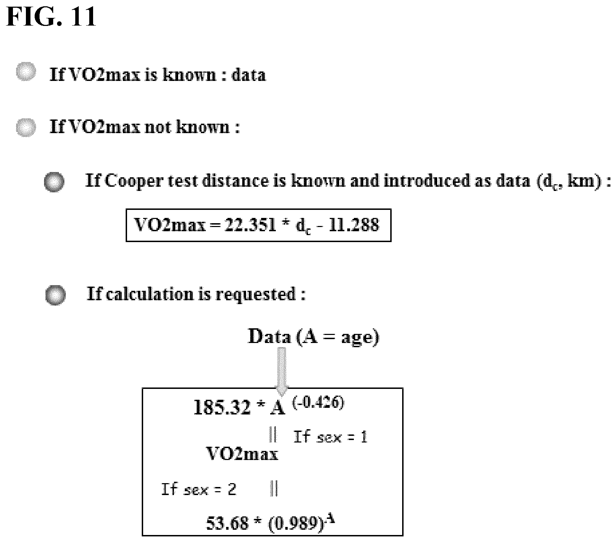

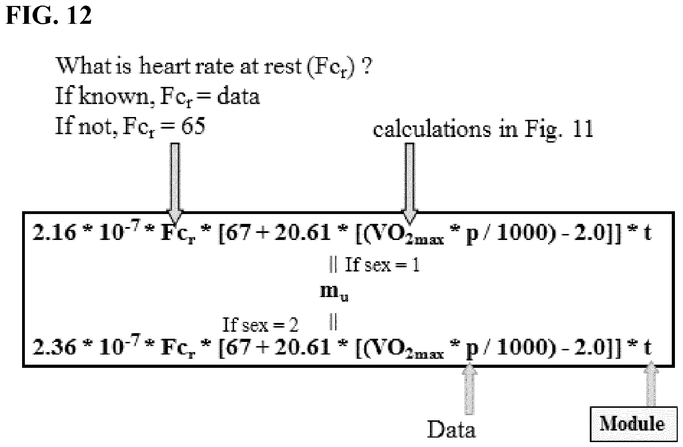

**FIG. 11** generally illustrates an embodiment of calculations for estimating the $VO_2$ max. The resultant $VO_2$ max can be used in the calculation shown in **FIG. 12** which estimates the volume of urine lost during effort ($m_u$) in liters.

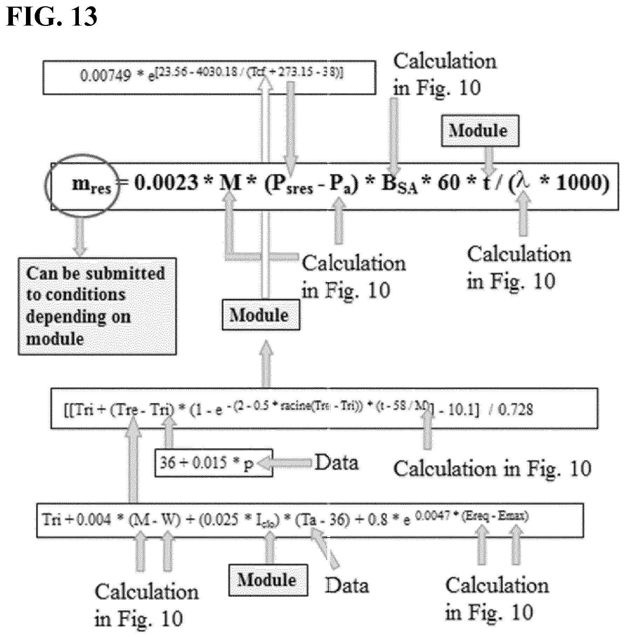

**FIG. 13** generally illustrates an embodiment of calculations for estimating the volume of water lost by breathing during effort ($m_{res}$) in liters.

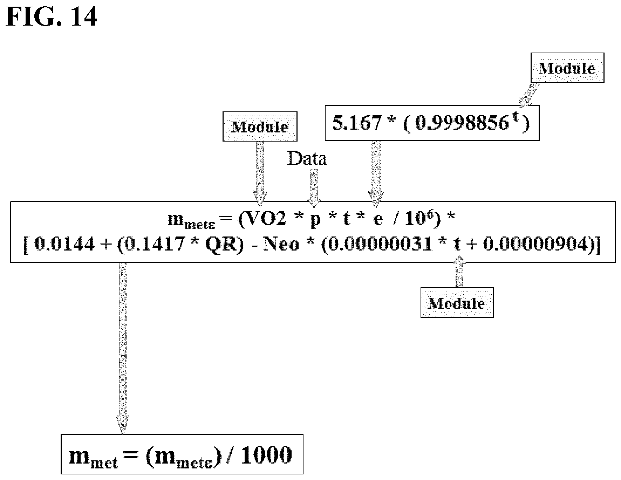

**FIG. 14** generally illustrates an embodiment of calculations for estimating the volume of water supplied by the body ($m_{met}$) in liters in one or more embodiments of the methods provided by the present disclosure. Preferably the volume of water synthesized in the body (e.g., by oxidation) during the effort ($m_{met\varepsilon}$) in liters is used to determine the volume of water supplied by the body ($m_{met}$) in liters.

The following calculations can be used to determine the glucose ratio supplied by neoglucogenesis (Neo), and then Neo can be used to determine the volume of water supplied by the body ($m_{met}$) as shown in **FIG. 14.**

$$\text{If } t \leq 90, \text{Neo} = (0.0806 * t + 2.33) + 16.99$$

$$\text{If } 90 \leq t \leq 240, \text{Neo} = 1.1643 * (0.0806 * t + 2.33) + 13.943$$

$$\text{If } t > 240, \text{Neo} = 40.5$$

The total of water needs during effort in liters ($m_{eau}$) can then be determined by adding the volume of sweat loss during effort in liters ($m_s$) as determined in the calculations shown in **FIG. 10,** the volume of urine loss during effort in liters ($m_u$) as determined in the calculations shown in **FIG. 12,** and the volume of water lost by breathing during effort in liters ($m_{res}$) as determined in the calculations shown in **FIG. 13,** and then subtracting the volume of water supplied by the body during effort in liters ($m_{met}$) as determined in the calculations shown in **FIG. 14.**

$$m_{eau} = m_s + m_u + m_{res} - m_{met}$$

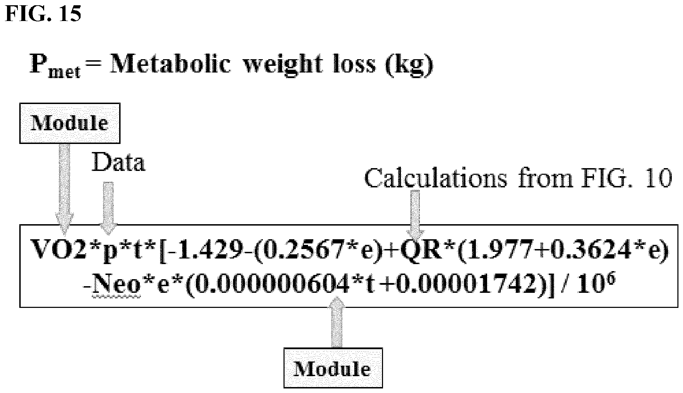

As shown in in **FIG. 15,** in some embodiments the metabolic weight loss in kg ($P_{met}$) can be determined. Then the metabolic weight loss in kg ($P_{met}$) can be used to determine the total body weight loss ($P_p$). For example, the water deficit after the training session or match ($m_b$) can be determined as follows:

$$m_b = m_s + m_u + m_{res} - m_{met} - V_e$$

Then $P_p = (m_b * 100) / (p - p_{met})$.

In an embodiment, mineral losses can be determined. For example, sodium losses in grams ($m_{Na}$) can be calculated as follows:

If the individual practices a regular training, $m_{Na} = Ar_{dc} \{0.66 * m_s + 1.035 * m_u\}$
If the individual does not practice a regular training, $m_{Na} = Ar_{dc} \{1.15 * m_s + 1.035 * m_u\}$
Golf is automatically considered a regular training. $Ar_{dc}$ is rounded to the upper ten.
Calcium losses in mg ($m_{Ca}$) can be determined by $m_{Ca} = 60 * m_s$.
Magnesium losses in mg ($m_{Mg}$) can be determined by $m_{Mg} = 24 * m_s$.

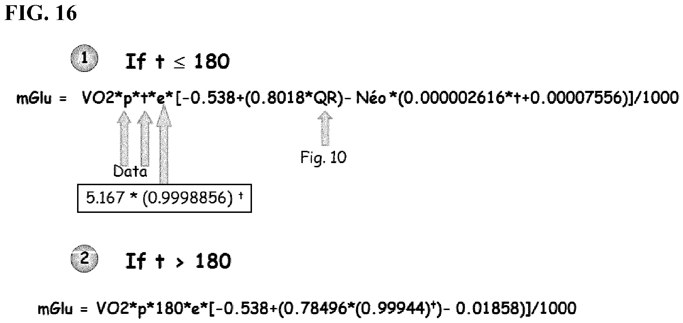

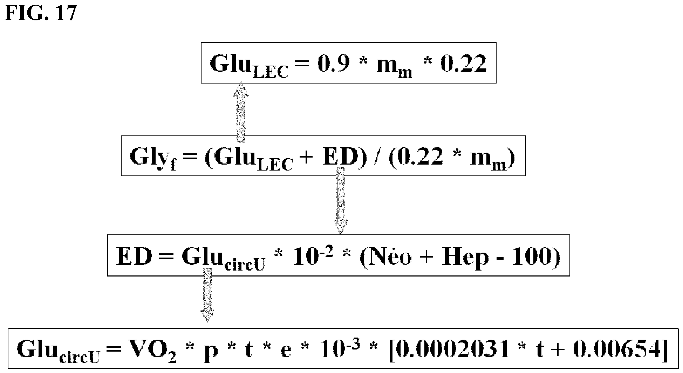

As shown in **FIG. 16,** in some embodiments the glucose losses in g ($m_{Glu}$) can be determined. As shown in **FIG. 17,** in some embodiments the blood sugar level after effort in g/L ($Gly_f$) can be determined. The blood sugar level after effort in g/L ($Gly_f$) can be determined using the lean mass of the individual ($m_m$) in kg which in turn can be determined from the fat mass ($m_g$) in kg and total weight (p) in kg as follows:

For a male, $m_g = (0.23 * p) - (20.15 * (h/100)) + 30.13$
For a female, $m_g = (0.47 * p) - (48.54 * (h/100)) + 75.06$

$$m_m = p - m_g$$

In an embodiment, gastric tolerance can be determined, and the amount of intake of water, minerals and/or glucose and/or the frequency of intake of water, minerals and/or glucose in the optimal personalized hydration plan can be based on the estimated gastric tolerance.

Accordingly, an aspect of the present disclosure is a method of decreasing or preventing dehydration in an individual from an athletic training session or match. Another aspect is a method of improving athletic performance of an individual in an athletic training session or match.

The methods can comprise accepting user input into an application provided by a device comprising a processor, the user input comprising information selected from the group consisting of a characteristic of the individual, a characteristic of the training session or match, and a combination thereof.

The characteristic of the individual can be selected from the group consisting of weight, height, gender, heart rate at rest, maximal heart rate, $VO_2$ max, effort intensity for the training session or match, a type of clothes worn during the training session or match, and combinations thereof. The characteristic of the training session or match is selected from the group consisting of a specific sport, a time duration, a position played by the individual, a location, an ambient temperature at the location, a humidity at the location, a wind speed at the location, and combinations thereof. Preferably at least a portion of the user input (e.g., at least a portion of the information) is accepted before the training session or match, most preferably the entirety of the user input.

The device providing the application can analyze the information provided by the user input to determine a water loss by the individual in the athletic training session or match. The analyzing of the information to determine the water loss can comprise determination of one or more (preferably all) of (i) water loss in sweat by the individual in the athletic training session or match, (ii) water loss in urine by the individual in the athletic training session or match, (iii) respiratory water loss by the individual in the training session or match, (iv) insensible water loss by the individual in the training session or match, (v) endogenic water formed by the individual in the training session or match, and (vi) a rate of gastric emptying by the individual. In some embodiments, the usual amount of water intake is included in the determination such that the water loss by the individual in the athletic training session or match determined by the application is the usual water loss by the individual in the athletic training session or match.

Preferably at least a portion of the analyzing is completed before the training session or match, most preferably the entirety of the analyzing. In an embodiment, the analyzing is performed automatically by the device and/or the application in response to the user input providing the information.

The device providing the application can display a personalized hydration plan that comprises an amount of water intake and/or a frequency of water intake, based on analysis of the information provided by the user input. For example, the personalized hydration plan (e.g., the amount of water intake and/or the frequency of water intake) can be based at least partially on one or more of the water loss in sweat, the water loss in urine, the respiratory water loss, the insensible water loss, the endogenic water, and the rate of gastric emptying. The amount of water intake and/or the frequency of water intake can be identified for consumption before the training session or match (e.g., one hour before until start), during the training session or match, and/or after the training session or match (e.g., the end until one hour after). In an embodiment, the creation and/or the display of the personalized hydration plan are performed automatically by the device and/or the application after the analyzing of at least a portion (preferably all) of the information.

In some embodiments, the method further comprises analyzing the information to determine a loss in the training session or match of at least one mineral selected from the group consisting of sodium, calcium and magnesium. In such embodiments, the at least one instruction (e.g., the amount of water intake and/or the frequency of water intake) further comprises an intake amount of the at least one mineral for the individual. The intake amount of the at least one mineral can be identified for consumption before the training session or match (e.g., one hour before until start), during the training session or match, and/or after the training session or match (e.g., the end until one hour after).

In some embodiments, the method further comprises analyzing the information to determine a glucose loss in the training session or match. In such embodiments, the at least one instruction (e.g., the amount of water intake and/or the frequency of water intake) further comprises an amount of glucose intake for the individual. The amount of glucose intake can be identified for consumption before the training session or match (e.g., one hour before until start), during the training session or match, and/or after the training session or match (e.g., the end until one hour after).

Preferably, consumption of water by the individual according to the personalized hydration plan will result in the individual having a hydration level at the end of the training session or match (or a time proximate to the end, such as one hour after the end or thirty minutes after the end) that is at least approximately equal to the hydration level at the start of the training session or match (or a time proximate to the beginning, such as one hour before the start or thirty minutes before the start).

Yet another aspect of the present disclosure is a method in which a usual amount of water intake by the individual in the training session or match is analyzed. The method can decrease or prevent dehydration in an individual from an athletic training session or match and/or improve athletic performance of an individual in an athletic training session or match.

For example, the individual can participate in one or more occurrences of the training session or match, and the usual amount of water intake can be the average amount of water consumed before these one or more occurrences of the training session or match (e.g., one hour before until start), during these one or more occurrences of the training session or match, and/or after these one or more occurrences of the training session or match (e.g., the end until one hour after). Then the individual can analyze the usual amount of water intake by the individual before a subsequent occurrence of the training session or match.

Preferably, the subsequent occurrence of the training session or match is substantially similar to the one or more occurrences of the training session or match from which the usual amount of water intake was obtained. For example, a substantially similar occurrence of a training session or match has one or more of the same sport, the same time duration, the same location, the same ambient temperature, the same humidity, the same effort intensity, the same type of clothes worn, the same position played by the individual, and the same wind speed.

The method can comprise accepting user input into an application provided by a device comprising a processor, the user input comprising a usual amount of water intake by the individual in the training session or match and further comprising information selected from the group consisting of a characteristic of the individual, a characteristic of the training session or match, and a combination thereof.

The characteristic of the individual can be selected from the group consisting of weight, height, gender, heart rate at rest, maximal heart rate, $VO_2$ max, effort intensity for the training session or match, a type of clothes worn during the training session or match, and combinations thereof. The characteristic of the training session or match is selected from the group consisting of a specific sport, a time duration, a position played by the individual, a location, an ambient temperature at the location, a humidity at the location, a wind speed at the location, and combinations thereof. Preferably at least a portion of the user input (e.g., at least a portion of the information) is accepted before the training session or match, most preferably the entirety of the user input.

The device providing the application can analyze the user input to determine a water loss by the individual in the athletic training session or match. The analyzing of the information to determine the water loss can comprise determination of one or more (preferably all) of (i) water loss in sweat by the individual in the athletic training session or match, (ii) water loss in urine by the individual in the athletic training session or match, (iii) respiratory water loss by the individual in the training session or match, (iv) insensible water loss by the individual in the training session or match, (v) endogenic water formed by the individual in the training session or match, and (vi) a rate of gastric emptying by the individual. In some embodiments, the usual amount of water intake is included in the determination such that the water loss by the individual in the athletic training session or match determined by the application is the usual water loss by the individual in the athletic training session or match.

Preferably at least a portion of the analyzing is completed before the training session or match, most preferably the entirety of the analyzing. In an embodiment, the analyzing is performed automatically by the device and/or the application in response to the user input providing the information.

The device providing the application can display an output on the device. When the water loss from the usual amount of water intake by the individual in the training session or match is less than a threshold, the output preferably is a first output that is a confirmation that the usual amount of water intake by the individual in the training session or match is sufficient to prevent dehydration in the training session or match. When the water loss from the usual amount of water intake by the individual in the training session or match is more than the threshold, the output preferably is a second output comprising a personalized hydration plan that comprises at least one instruction selected from the group consisting of an amount of water intake and a frequency of water intake, and the at least one instruction is based at least partially on the water loss determined from the information. In an embodiment, the display of the output is performed automatically by the device and/or the application after the analyzing of at least a portion (preferably all) of the information.

The personalized hydration plan (e.g., the amount of water intake and/or the frequency of water intake) can be based at least partially on one or more of the water loss in sweat, the water loss in urine, the respiratory water loss, the insensible water loss, the endogenic water, and the rate of gastric emptying. The amount of water intake and/or the frequency of water intake can be identified for consumption before the training session or match (e.g., one hour before until start), during the training session or match, and/or after the training session or match (e.g., the end until one hour after).

Preferably, consumption of water by the individual according to the personalized hydration plan will result in the individual having a hydration level at the end of the training session or match (or a time proximate to the end, such as one hour after the end or thirty minutes after the end) that is at least approximately equal to the hydration level at the start of the training session or match (or a time proximate to the beginning, such as one hour before the start or thirty minutes before the start).

In some embodiments of the methods disclosed herein, the method further comprises administering water to the individual according to the personalized hydration plan.

In any of the embodiments disclosed herein, the program (e.g., an application) can be executed by a device of a user who is preferably the individual participating in the athletic training session or match or an individual involved in providing hydration at the athletic training session or match. The device can be a stationary and/or mobile communications device, for example at least one of a mobile telephone, such as a smartphone; a laptop computer; a desktop personal computer; a tablet; or a personal digital assistant. The program can be executed and/or stored by the device. The program can be obtained by the device using wired and/or wireless networks, for example the internet, telephone lines, WiFi/WLAN, 3G networks, or the like.

The device can store the program in a non-transitory computer readable storage medium that is any computer-readable media except for a transitory, propagating signal. Non-limiting examples of computer-readable media that can store the program include any type of disk including optical disks, CD-ROMs, and magnetic-optical disks; read-only memories (ROMs); random access memories (RAMs); EPROMs; EEPROMs; magnetic or optical cards; program specific integrated circuits (ASICs); and any type of media suitable for storing electronic instructions.

The program can be obtained and/or can be accessed by selection of a corresponding icon using the device. The icon can visually indicate the identity of the program, such as by depicting a representation of water, a beverage, or a beverage container. In an embodiment, the program can be obtained from an application store for the corresponding type of device or operating system of the device. In an embodiment, the program can be accessed using a web browser and the internet.

The device providing the application can provide a user interface that can accept information and display information. The user can enter the information into the data fields using any known input component of the device providing the application, such as a keyboard, a touchscreen, a trackball, and the like, and the present disclosure is not limited to a specific embodiment of the input component of the device.

The application can request that the user enter into the user interface information regarding the individual and their training session or match, for example one or more of the date of the training session or match, the time of the training session or match, the humidity of the training session or match, the location of the training session or match, the age of the individual, the weight of the individual, the duration of the training session or match, the heart rate at rest of the individual, the $VO_2$ max of the individual, and how much water the individual usually consumes during the training session or match Then the application can analyze the information, for example using the calculations detailed above, and display the calculated water losses, e.g., one or more of $m_u$, $m_s$, $m_{res}$ and $m_{eau}$ and/or the calculated water intake, e.g., one or more of $m_{met}$ and $V_e$. In a preferred embodiment, the application displays the water deficit of the individual at the end of the training session or match using their usual hydration and/or the weight loss of the individual at the end of the training session or match using their usual hydration.

The application can confirm that the usual hydration is acceptable, for example if their estimated weight loss is about zero. Additionally or alternatively, the application can provide an optimal personalized hydration plan and can display the expected weight loss of the individual at the end of the training session or match based on the hydration plan, which preferably is less than the weight loss using their usual hydration.

In an embodiment, the application displays the typical effect of the weight loss from the usual hydration. For example, if the weight loss from the usual hydration is less than 1 %, the application can display that there is no detrimental effect and performance is maximal; if the weight loss from the usual hydration is less than 1%, the application can display that there is no detrimental effect and performance is decreased; if the weight loss from the usual hydration is 1-2 %, the application can display that there is an onset of thirst and performance is decreased; if the weight loss is 2-3 %, the application can display that there is a strong thirst and performance is decreased 3-10 %; if the weight loss is 3-4 %, the application can display that there is dry mouth and dark urine and performance is decreased by 5-20 %; if the weight loss from the usual hydration is 4-6 %, the application can display that there is difficulty concentrating and a risk of headache and performance is decreased by 20-30 %; and if the weight loss is 6-8 %, the application can display that there is tingling and numbness in the extremities and a risk of collapse and performance is decreased by at least 30 %.

The application can display an optimal personalized hydration plan that identifies an amount of water to drink before the training session or match and/or a frequency of intake; identifies an amount of water to drink during the training session or match and/or a frequency of intake, e.g., once every fifteen minutes; and/or identifies an amount of water to drink after the training session or match and/or a frequency of intake, e.g., according to the thirst of the individual.

The application can identify the expected weight loss (if any) if the hydration plan is followed by the individual; the ratio of the water volume proposed by the hydration plan to the water volume typically consumed by the individual; and a message to increase gradually if the ratio is large (e.g., more than 1.2).

In an embodiment, the application can identify other losses of the individual at the end of the training session or match using their usual hydration, for example one or more of $m_{Ca}$, $m_{Mg}$, $m_{Na}$, $m_{Glu}$ and $m_{Cal}$. The hydration plan can include a suggestion to include a suggested amount of one or more of calcium, magnesium, sodium, glucose or calories; and the individual can ensure that these are included in beverages consumed before, during and/or after the training session or match, for example by adding at least a portion of the suggested amount in water consumed before, during and/or after the training session or match.

Data fields for the user to input the information can be displayed on one or more screens of the user interface, and the results of the analysis thereof can be displayed on one or more same screens and/or one or more different screens.

Other information and criteria not known to be related to dehydration at the time of this patent application but later discovered to be a factor can be used by the application, and the present disclosure is not limited to the above examples of the information and the criteria. The information and the criteria can be any information and criteria found to be related to loss of hydration, e.g., dehydration, in an individual participating in a training session or match.

In some embodiments, the device providing the application preferably creates a profile for the user such that the profile is stored and can be edited using the user interface. The device providing the application can store at least a portion of the profile. Alternatively or additionally, a remotely located database can store at least a portion of the profile. In an embodiment, the remotely located database is connected to the device by a wireless network, such as the internet.

Accordingly, another aspect of the present disclosure is a device configured to perform at least part of one or more of the methods disclosed herein.

EXAMPLE

The following non-limiting example is illustrative of optimal personalized hydration in an individual participating in a handball training session or match, in one or more embodiments provided by the present disclosure. The values discussed in this example can easily be determined for other sports.

The estimated volume of sweat loss ($m_{si}$) can be determined using the calculation discussed above and shown in FIG. 10. In an embodiment, the value of final skin temperature ($T_{pf}$) used in the calculation of $m_{si}$ can be determined as follows:

$$T_{pf} = 0.403 * T_a + 21.46$$

In an embodiment, the value of the clothes thermal resistance coefficient in $°C.m^2.W^{-1}$ ($I_{clo}$) used in the calculation of $m_{si}$ can be determined as follows:

$$I_{clo} = 0.74 * [0.278 + 0.004 * (M\text{-}105)]^{-0.28}$$

In an embodiment, the value of the clothes permeability index (no units) ($I_m$) used in the calculation of $m_{si}$ can be determined as follows:

$$I_m = 0.94 * [0.278 + 0.004 * (M\text{-}105)]^{-0.28}$$

In this non-limiting example, a = 35.09 and b = 0.559 for the calculation of $m_{si}$.

Then the estimated volume of sweat loss ($m_{si}$) can be adjusted based on whether the effort was a training session or a match and/or the intensity of the effort. For example, if the individual is participating in a handball match, the estimated sweat loss ($m_s$) can be divided by 1.239; and if the individual is participating in a handball training session at low intensity, medium intensity or high intensity, the estimated sweat loss ($m_s$) can be divided by 1.61, 1.18 and 0.989 respectively.

In some embodiments, the water deficit after the training session or match ($m_b$) can be predicted. For example, the water deficit after a handball training session can be estimated by $m_b = 0.8951 * V_e + 0.2376 - m_u - m_{res}$; and the water deficit after a handball match can be estimated by $m_b = 0.8491 * V_e + 0.5005 - m_u - m_{res}$.

In some embodiments, a final estimate of volume of sweat lost during the effort ($m_{sf}$) is calculated by comparing the initial estimate ($m_s$) to the water deficit after the training session or match ($m_b$) and requesting that the individual indicate their extent of sweating, e.g., "normal," "low" or "high." For example, in a handball training session or match:

(i) if $m_s \geq 1.25 * m_b$: $m_{sf} = m_s$ if sweating is normal; $m_{sf} = (m_s + m_b)/2$ if sweating is low; and $m_{sf} = m_s$ if sweating is high

(ii) if $m_s \leq 0.833 * m_b$: $m_{sf} = m_s$ if sweating is normal; and $m_{sf} = m_s$ if sweating is low; and $m_{sf} = (m_s + m_b)/2$ if sweating is high

(iii) if $0.833 * m_b \leq m_s \leq 1.25 * m_b$: $m_{sf} = m_s$

In an embodiment, the value of the oxygen consumed during the effort ($VO_2$), which is then used in the calculation of $m_s$ shown in **FIG. 10,** can be determined as follows (BMR calculation coming from literature data and not original equations):

For a male handball fielder in a training session of intensity level 1, $VO_2 = 29.6 - (281.5 / p)$

For a male handball fielder in a training session of intensity level 2, $VO_2 = 37.3 - (388.1 / p)$

For a male handball fielder in a training session of intensity level 3, $VO_2 = 44.0 - (570.2 / p)$

For a male handball goalie in a training session of intensity level 1, $VO_2 = 29.6 - (281.5 / p)$

For a male handball goalie in a training session of intensity level 2, $VO_2 = 33.6 - (349.3 / p)$

For a male handball goalie in a training session of intensity level 3, $VO_2 = 39.6 - (513.2 / p)$

For a male handball fielder in a match, $VO_2 = (30 * VO_2(1) + (t' * VO_2(2)) + (70 - t') * VO_2(3))/100$, where $VO_2(1) = 0.45 * VO_2 \text{ max}$; $VO_2(2) = 0.65 * VO_2 \text{ max}$; and $VO_2(3) = 0.35 * VO_2 \text{ max}$

For a male handball goalie in a match, $VO_2 = (30 * VO_2(1) + (t' * VO_2(2)) + (70 - t') * VO_2(3))/100$, where $VO_2(1) = 0.45 * VO_2 \text{ max}$; $VO_2(2) = 0.50 * VO_2 \text{ max}$; and $VO_2(3) = 0.25 * VO_2 \text{ max}$

For a female handball player, $VO_2 = VO_2 \text{ (male)} * BMR2 / BMR1$, where a female from 10 years to 18 years of age has a $BMR1 = (17.5 * p + 651) / 1440$ and a $BMR2 = (12.2 * p + 746) / 1440$; a female from 19 years to 30 years of age has a $BMR1 = (15.3 * p + 679) / 1440$ and a $BMR2 = (14.7 * p + 496) / 1440$; a female from 31 years to 60 years of age has a $BMR1 = (11.6 * p + 879) / 1440$ and a $BMR2 = (8.7 * p + 829) / 1440$; and a female over 60 years of age has a $BMR1 = (13.5 * p + 487) / 1440$ and a $BMR2 = (10.5 * p + 596) / 1440$.

For the above calculations, the $VO_2$ max can be estimated if not known. For example, the $VO_2$ max of an amateur female individual can be estimated as 40 mL.min$^{-1}$.kg$^{-1}$, the $VO_2$ max of an elite female individual can be estimated as 45 mL.min$^{-1}$.kg$^{-1}$.the $VO_2$ max of an amateur male individual can be estimated as 50 mL.min$^{-1}$.kg$^{-1}$, and the $VO_2$ max of an elite male individual can be estimated as 55 mL.min$^{-1}$.kg$^{-1}$.

The volume of urine loss during the effort ($m_u$) can be calculated as shown in **FIG. 12**. The volume of respiratory loss during the effort ($m_{res}$) can be calculated as shown in **FIG. 13**. In this non-limiting example, $T_{rf} = [[T_{ri} + (T_{re} - T_{ri}) * 1 - e^{-(2 - 0.5 * \text{root}(Tre-Tri)) * (t - 58 / M)}] - 10.1] / 0.728$. In turn, $T_{cf} = 0.8 * T_{rf} + 0.2 * T_{pf}$. Then $T_{cf}$ can be used to calculate $P_{sres}$ as shown in **FIG. 13.**

The volume of water supplied by the body during the effort ($m_{met}$) can be determined by adding the volume of water synthesized in the body (e.g., by oxidation) during the effort ($m_{met\varepsilon}$) to the water released from glycogen in the body ($m_{metglyco}$):

$$m_{met} = m_{met\varepsilon} + m_{metglyco}$$

For example, as set forth in the literature, $m_{met\varepsilon}$ can be calculated by $m_{met\varepsilon} = (VO_2 * p * t * e * 10^{-6}) * (0.0144 + (0.1417 * QR) - \text{Neo} * (0.00000031 * t + 0.00000904))$. Also, $m_{metglyco}$ can be calculated by $m_{metglyco} = (VO_2 * p * t * e * 10^{-6}) * (-1.417 + (2.0012 * (0.99944)^t) - (0.000494 * t) - \text{Neo} * (0.000000975 * t + 0.0000313) + \text{Hep} * (0.00000591 * t + 0.000191))$.

The glucose ratio supplied by gluconeogenesis (%) (Neo) can be calculated as established in the literature and set forth above. Specifically, if $t \leq 90$, $\text{Neo} = (0.0806 * t + 2.33) + 16.99$; if $90 \leq t \leq 240$, $\text{Neo} = 1.1643 * (0.0806 * t + 2.33) + 13.943$; and if $t > 240$, $\text{Neo} = 40.5$.

The glucose ratio provided by liver glycogen (Hep) can be calculated as established in the literature, specifically: if $t \leq 60$, $\text{Hep} = (0.0806 * t + 2.33) + 577.37$; if $60 \leq t \leq 240$, $\text{Hep} = -1.314 * (0.0806 * t + 2.33) + 87.073$; and if $t > 240$, $\text{Hep} = 57$.

Then the total water loss during the effort ($m_{eau}$) can be calculated: $m_{eau} = m_{sf} + m_u + m_{res} - m_{met}$.

The metabolic weight loss in kg ($P_{met}$) can be determined as shown in **FIG. 15.**

Mineral losses can be determined as detailed above. For example, sodium losses in g ($m_{Na}$) can be calculated by $m_{Na} = 0.66 * m_s + 1.035 * m_u$, calcium losses in mg ($m_{Ca}$) can be determined by $m_{Ca} = 60 * m_s$, and magnesium losses in mg ($m_{Mg}$) can be determined by $m_{Mg} = 24 * m_s$.

The calorie expenditure can be determined by $m_{Cal} = VO_2 * p * e * t / 1000$.

The fat mass ($m_g$) and the lean mass ($m_m$) in kg can be determined using weight in kg (p). For a male, if 0.23 * p - 20.15 * (h /100) + 30.13 < 6, then fat mass ($m_g$) = 6 * p /100; and if 0.23 * p - 20.15 * (h /100) + 30.13 $\geq$ 6, then fat mass ($m_g$) = (0.23 * p - 20.15 * (h /100) + 30.13) * p / 100. For a female, if 0.47 * p - 48.54 * (h /100) + 75.06 < 6, then fat mass ($m_g$) = 6 * p /100; and if 0.47 * p - 48.54 * (h /100) + 75.06 $\geq$ 6, then fat mass ($m_g$) = (0.47 * p - 48.54 * (h /100) + 75.06) * p / 100.

Then lean mass ($m_m$) = p - $m_g$.

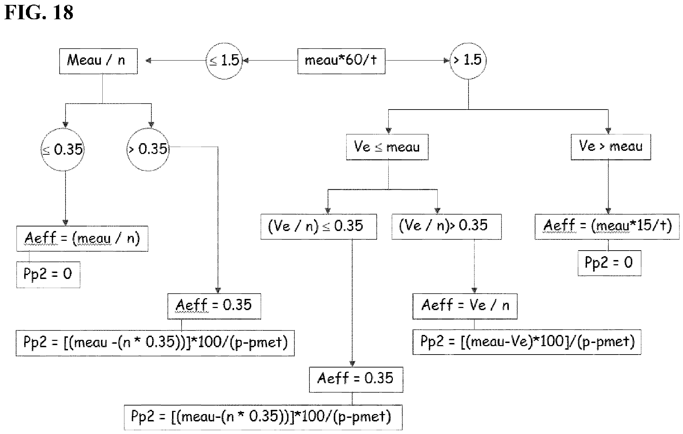

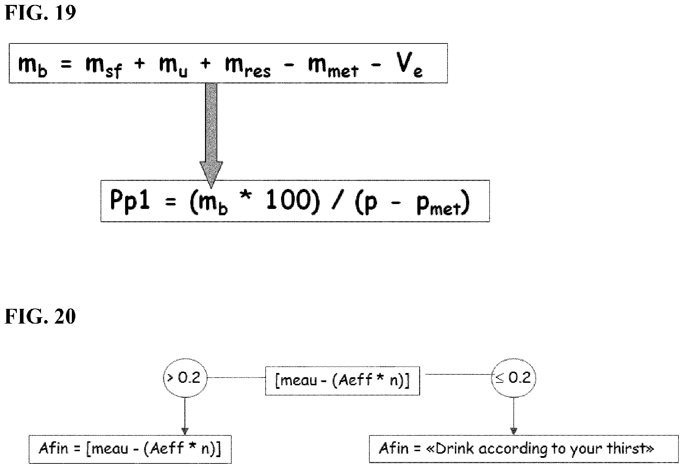

As non-limiting examples of further calculations utilizing those disclosed above, **FIG. 18** generally illustrates the volume of water to ingest at each break depending on water losses and individual gastric tolerance. **FIG. 19** generally illustrates calculations of water deficit after the training session or match ($m_b$) and the subsequent calculation of total body weight loss ($P_{p1}$). **FIG. 20** generally illustrates the volume of water to drink in addition to the usual volume drunk by the subject. In this regard, the difference between water needs and water usually drunk is calculated; if this difference is lower than 0.2 L, the subject is instructed to drink according to their thirst, otherwise the proposed volume is suggested for consumption by the subject.

<u>Abbreviations</u>

| | |
|---|---|
| A | Age (years old) |
| BSA | Body surface ($m^2$) |
| C | Heat exchange by convection ($W.m^{-2}$) |
| Cal | Average caloric expenditure for the effort ($kCal.min^{-1}$) |
| $C_{res}$ | Respiratory dry heat ($W.m^{-2}$) |
| $d_c$ | Cardiac output ($L.min^{-1}$) |
| $E_{max}$ | maximal evaporative capacity ($W.mmHg^{-1}.m^{-2}$) |
| $E_{req}$ | required evaporative cooling ($W.°C^{-1}.m^{-2}$) |
| $E_{res}$ | Respiratory heat loss ($W.m^{-2}$) |
| e | Oxygen energetic equivalent ($kcal.L^{-1}$) |
| $F_{cf}$ | Final heart rate (bpm) |
| $F_{ci}$ | Initial heart rate (bpm) |
| $Gly_f$ | Final glycemia ($g.L^{-1}$) |
| h | Height (m) |
| $H_a$ | Ambient moisture (%) |
| $H_p$ | Skin moisture (%) |
| Hep | Glucose ratio supplied by liver glycogen (%) |
| I | Effort intensity (%) |
| $I_{clo}$ | Clothes thermal resistance coefficient $°C.m^2.W^{-1}$ |
| $I_m$ | Clothes permeability index |
| M | Metabolic heat ($W.m^{-2}$) |
| $m_{Ca}$ | Calcium losses (mg) |
| $m_{eau}$ | Total water needs (L) |
| $m_g$ | Fat mass (kg) |
| $m_{Glu}$ | Glucose losses (g) |
| $m_m$ | Lean mass (kg) |
| $m_{Na}$ | Sodium losses (g) |
| $m_{Mg}$ | Magnesium losses (mg) |
| $m_{met}$ | Metabolic water (volume of water supplied by the body) (L) |
| $m_{met\varepsilon}$ | Water synthesized by the body (L) |
| $m_s$ | Sweat losses (L) |
| $m_u$ | Urine losses (L) |
| $m_{res}$ | Respiratory losses (L) |
| Neo | Glucose ratio supplied by neoglucogenesis (%) |
| p | Weight (kg) |
| $P_a$ | Ambient water vapor pressure (mmHg) |
| $P_{sa}$ | Ambient saturated water vapor pressure (mmHg) |
| $P_p$ | Weight loss (Pp1, Pp2) (%) |
| $P_{sp}$ | Skin saturated water vapor pressure (mmHg) |
| $P_{sres}$ | Respiratory tract saturated water vapor pressure (mmHg) |
| QR | Respiratory quotient (no units) |
| R | Heat exchange by radiation ($W.m^{-2}$) |

| | |
|---|---|
| R + C | Combined dry heat ($W.m^{-2}$) |
| t | Length of effort (min) |
| $T_a$ | Dry ambient temperature (°C) |
| $T_{ci}$ | Initial body temperature (°C) |
| $T_{cf}$ | Final body temperature (°C) |
| $T_{pf}$ | Final skin temperature (°C) |
| $T_{pi}$ | Initial skin temperature (°C) |
| $T_{re}$ | Equilibrium rectal temperature (°C) |
| $T_{rf}$ | Final rectal temperature (°C) |
| $T_{ri}$ | Initial rectal temperature (°C) |
| $VO_2$ max | Maximal oxygen consumption ($mL.min^{-1}.kg^{-1}$) |
| $V_e$ | Volume usually consumed during effort (L) |
| W | Mechanical work done ($W.m^{-2}$) |
| w | Index of skin humidification by sweat (no units) |
| $\lambda$ | Vapor latent heat ($J.g^{-1}$) |
| $\lambda'$ | Corrective factor to vapor latent heat ($J.g^{-1}$) |
| $\lambda f$ | Final vapor latent heat ($J.g^{-1}$) |
| $\eta$ | Sweating efficacy (no units) |

**Claims**

1. An athletic training dehydration prevention system for decreasing or preventing dehydration in an individual from an athletic training session or match comprising:

   a hydration input module configured to receive at least one user input representing information selected from the group consisting of a characteristic of the individual, a characteristic of the training session or match, and a combination thereof;
   a hydration calculation module comprising at least one processor and at least one memory device that stores a plurality of instructions which, when executed by the at least one processor, (a) analyze the information provided by the user input to determine a water loss by the individual in the athletic training session or match based on the determination of total water loss by adding a water loss from sweat, a water loss in urine, a respiratory water loss, an insensible water loss, and subtracting an endogenic water formed during the athletic training session or match, and (b) determine a personalized hydration plan for the individual based on the user input; and
   an output module configured to cause a display device to display aspects of the personalized hydration plan, including at least one selected from the group consisting of: a recommended amount of water intake for the individual based on the determined water loss and a frequency of water consumption based on determined water loss and wherein at least a portion of the amount of water intake is identified for consumption before and/or during and/or after the training session or match,
   wherein :

   - the water loss from sweat is

   $$S = (60 * a * E_{req} * E_{max}^{-b} * t * Bsa) / \lambda f$$

   with $E_{req}$ = required evaporative cooling ($W.°C^{-1}.m^{-2}$), $E_{max}$ = maximal evaporative capacity ($W.mmHg^{-1}.m^{-2}$), $\lambda f$ = final vaporization heat ($J.g^{-1}$), Bsa = Body surface ($m^2$) and t=length of effort (min)
   - the water loss in urine is

   $$U = a * 10^{-7} * HRr * (67+20.61 * ((VO_2 \text{ max} * weight/1000)-2.0)) * t$$

   with a=2.16 for male and a=2.36 for female, t = length of effort (min), HRr = heart rate at rest (bpm), $VO_2$ max = maximal consumption of oxygen ($mL.min^{-1}.kg^{-1}$)
   - the respiratory water loss is

   $$R = 0.0023 * M * (P_{sres}-P_a) * Bsa * 60 * t/\lambda$$

with M = metabolic heat (W.m$^2$), $P_{sres}$ = saturated water vapor pressure of respiratory tract (mmHg), $P_a$ = ambient water vapor pressure (mmHg), Bsa = Body surface (m$^2$), λ = vaporization latent heat (J.g$^{-1}$), t=length of effort (min)

- the insensible water loss is,

$$I_n = (6.0 + 1.75 * (P_{sp} - P_a)/7.5) * Bsa * t$$

with $P_{sp}$ = skin water vapor pressure (mmHg), $P_a$ = ambient water vapor pressure (mmHg), Bsa = Body surface (m$^2$), t=length of effort (min), and

- the endogenic water formed is

$$m_{met} = m_{metε} + m_{metglyco}$$

with $m_{metε}$ = (VO$_2$ * p * t * e * 10$^{-6}$) * (0.0144 + (0.1417 * QR) - Neo * (0.00000031 * t + 0.00000904)), and $m_{metglyco}$ = (VO$_2$ * p * t * e * 10$^{-6}$) * (-1.417 + (2.0012 * (0.99944)$^t$) - (0.000494 * t) - Neo * (0.000000975 * t + 0.0000313) + Hep * (0.00000591 * t + 0.000191)), with VO$_2$ = oxygen consumption (mL.min$^{-1}$.kg$^{-1}$), p=weight (kg), t= length of effort (min), e= oxygen energetic equivalent (kcal.L$^{-1}$), QR= respiratory quotient (no units), Neo= glucose ratio supplied by neoglucogenesis (%), Hep= glucose ratio supplied by liver glycogen (%)

2. The athletic training dehydration prevention system of Claim 1 wherein the characteristic of the individual is selected from the group consisting of weight, height, gender, heart rate at rest, maximal heart rate, VO$_2$ max, effort intensity for the training session or match, a type of clothes worn during the training session or match, and combinations thereof.

3. The athletic training dehydration prevention system of Claim 1 or 2 wherein the characteristic of the training session or match is selected from the group consisting of a specific sport, a time duration, a position played by the individual, a location, an ambient temperature at the location, a humidity at the location, a wind speed at the location, and combinations thereof.

4. The athletic training dehydration prevention system of any of the preceding Claims wherein the user input comprises time duration of the training session or match, weight of the individual, height of the individual, age of the individual, gender of the individual, ambient temperature at the location of the training session or match, and optionally wind speed at the location of the training session or match, and wherein the plurality of instructions cause the hydration calculation module to determine the water loss in sweat by the individual is based at least partially on the data.

5. The athletic training dehydration prevention system of any of the preceding Claims wherein the user input comprises a time duration of the training session or match, heart rate at rest of the individual, age of the individual, and optionally VO$_2$ max of the individual, and wherein the plurality of instructions cause the hydration calculation module to determine the water loss in urine by the individual is based at least partially on the data.

6. The athletic training dehydration prevention system of any of the preceding Claims wherein the user input comprises a time duration of the training session or match, weight of the individual, height of the individual, age of the individual, gender of the individual, ambient temperature at the location of the training session or match, and optionally wind speed at the location of the training session or match, and wherein the plurality of instructions cause the hydration calculation module to determine the respiratory water loss by the individual is based at least partially on the data.

7. The athletic training dehydration prevention system of any of the preceding claims wherein the user input comprises a time duration of the training session or match, weight of the individual, height of the individual, and ambient temperature at the location of the training session or match, and wherein the plurality of instructions cause the hydration calculation module to determine the insensible water loss is based at least partially on the data.

8. The athletic training dehydration prevention system of the preceding Claims wherein the plurality of instructions cause the hydration calculation module to determine an initial skin temperature of the individual and a final skin temperature of the individual for the training session or match and to determine the personalized hydration plan based at least partially on the initial and final skin temperatures.

9. The athletic training dehydration prevention system of the preceding Claims wherein the user input comprises a time duration of the training session or match, weight of the individual, age of the individual, and gender of the individual, and wherein the plurality of instructions cause the hydration calculation module to determine the amount of endogenic water formed by the individual based at least partially on the user input.

10. The athletic training dehydration prevention system of any of the preceding Claims, wherein the plurality of instructions case the hydration calculation module to analyze the user input to determine a rate of gastric emptying by the individual and to determine a recommended frequency of water intake based at least partially on the rate of gastric emptying by the individual.

11. The athletic training dehydration prevention system of any of the preceding Claims, wherein the plurality of instructions cause the hydration calculation module to analyze the user input to determine a loss in the training session or match of at least one mineral selected from the group consisting of sodium, calcium and magnesium and to determine a recommended amount of intake of the at least one mineral for the individual.

12. The athletic training dehydration prevention system of any of the preceding Claims, wherein the plurality of instructions cause the hydration calculation module to analyze the user input to determine a glucose loss in the training session or match and to determine a recommended amount of glucose intake for the individual.

13. The athletic training dehydration prevention system of the preceding Claims wherein the hydration input module is configured to receive the user input, and the hydration calculation module is configured to determine the personalized hydration plan, prior to the user participating in the training session or match.

14. The athletic training dehydration prevention system of any of the preceding Claims wherein the personalized hydration plan improves athletic performance in the individual during the training session or match.

15. The athletic training dehydration prevention system of any of the preceding Claims wherein the user input includes an identification of a specific sport, and the plurality of instructions cause the hydration calculation module to analyze the user input for a first sport using at least one variable or equation different than analysis of the user input for a different second sport even if the information is otherwise identical.

**Patentansprüche**

1. System zum Vorbeugen von Dehydratation bei sportlichem Training zum Verringern oder Vorbeugen von Dehydratation bei einer Person von einer sportlichen Trainingseinheit oder einem Wettkampf, umfassend:

ein Hydratationseingabemodul, das konfiguriert ist, um mindestens eine Benutzereingabe zu empfangen, die Informationen darstellt, die aus der Gruppe ausgewählt sind, bestehend aus einer Eigenschaft der Person, einer Eigenschaft der Trainingseinheit oder des Wettkampfs und einer Kombination davon;
ein Hydratationsberechnungsmodul, umfassend mindestens einen Prozessor und mindestens eine Speichervorrichtung, die eine Vielzahl von Anweisungen speichert, die, wenn sie durch den mindestens einen Prozessor ausgeführt werden, (a) die durch die Benutzereingabe bereitgestellten Informationen analysieren, um einen Wasserverlust der Person bei dem sportlichen Training oder dem Wettkampf zu bestimmen, basierend auf der Bestimmung eines Gesamtwasserverlusts durch Hinzufügen eines Wasserverlusts von Schweiß, eines Wasserverlusts in Urin, eines Wasserverlusts durch Atmung, eines unmerklichen Wasserverlusts und Subtrahieren eines während des sportlichen Trainings oder Wettkampfs ausgebildeten endogenen Wassers, und (b) einen personalisierten Hydratationsplan für die Person basierend auf der Benutzereingabe bestimmen; und
ein Ausgabemodul, das konfiguriert ist, um zu veranlassen, dass eine Anzeigevorrichtung Aspekte des personalisierten Hydratationsplans anzeigt, der mindestens eines einschließt, ausgewählt aus der Gruppe, bestehend aus: einer empfohlenen Wasseraufnahmemenge für die Person basierend auf dem bestimmten Wasserverlust und einer Häufigkeit eines Wasserkonsums basierend auf dem bestimmten Wasserverlust, und wobei mindestens ein Teil der Wasseraufnahmemenge für den Konsum vor und/oder während und/oder nach der Trainingseinheit oder dem Wettkampf identifiziert wird,
wobei:

- der Wasserverlust von Schweiß ist

$$S = (60 * a * E_{erf} * E_{max}^{-b} * t * Bsa) / \lambda f$$

wobei $E_{erf}$ = erforderliche Verdunstungskühlung (W.°C$^{-1}$.m$^{-2}$), $E_{max}$ = maximale Verdunstungskapazität (W.mmHg$^{-1}$.m$^{-2}$), $\lambda$f = endgültige Verdampfungswärme (J.g$^{-1}$), Bsa = Körperoberfläche (m$^2$) und t=Dauer der Anstrengung (min)
- der Wasserverlust in Urin ist

$$U = a * 10^{-7} * HRr * (67+20{,}61 * ((VO_2max * Gewicht/1000)-2{,}0)) * t$$

wobei a=2,16 für Männer und a=2,36 für Frauen, t = Dauer der Anstrengung (min), HRr = Ruheherzfrequenz (bpm), $VO_2$max = maximaler Sauerstoffverbrauch (ml.min$^{-1}$.kg$^{-1}$)
- der Wasserverlust durch Atmung ist

$$R = 0{,}0023 * M * (P_{sres}-P_a) * Bsa * 60 * t/\lambda$$

wobei M = metabolische Wärme (W.m$^2$), $P_{sres}$ = gesättigter Wasserdampfdruck der Atemwege (mmHg), $P_a$ = Umgebungswasserdampfdruck (mmHg), Bsa = Körperoberfläche (m$^2$), $\lambda$ = latente Verdampfungswärme (J.g$^{-1}$), t=Dauer der Anstrengung (min)
- der unmerkliche Wasserverlust ist,

$$I_n = (6{,}0 + 1{,}75 * (P_{sp} - P_a)/7{,}5) * Bsa * t$$

wobei $P_{sp}$ = Hautwasserdampfdruck (mmHg), $P_a$ = Umgebungswasserdampfdruck (mmHg), Bsa = Körperoberfläche (m$^2$), t=Dauer der Anstrengung (min) und
- das ausgebildete endogene Wasser ist

$$m_{met} = m_{met\varepsilon} + m_{metglyco}$$

wobei $m_{mete}$ = (VO$_2$ * p * t * e * 10$^{-6}$) * (0,0144 + (0,1417 * QR) - Neo * (0,00000031 * t + 0,00000904)) und $m_{metglyco}$ = (VO$_2$ * p * t * e * 10$^{-6}$) * (-1,417 + (2,0012 * (0,99944)$^t$) - (0,000494 * t) - Neo * (0,000000975 * t + 0,0000313) + Hep * (0,00000591 * t + 0,000191)), wobei VO$_2$ = Sauerstoffverbrauch (ml.min$^{-1}$.kg$^{-1}$), p=Gewicht (kg), t=Dauer der Anstrengung (min), e= Sauerstoff-Energieäquivalent (kcal.L$^{-1}$), QR= respiratorischer Quotient (keine Einheiten), Neo= durch Neoglucogenese bereitgestellter Glucoseanteil (%), Hep= durch Leberglycogen bereitgestellter Glucoseanteil (%)

2. System zum Vorbeugen von Dehydratation bei sportlichem Training nach Anspruch 1, wobei die Eigenschaft der Person aus der Gruppe ausgewählt ist, bestehend aus einem Gewicht, einer Größe, einem Geschlecht, einer Ruheherzfrequenz, einer maximalen Herzfrequenz, einer VO$_2$max, einer Anstrengungsintensität für die Trainingseinheit oder den Wettkampf, einer Art von Kleidung, die während der Trainingseinheit oder des Wettkampfs getragen wird, und Kombinationen davon.

3. System zum Vorbeugen von Dehydratation bei sportlichem Training nach Anspruch 1 oder 2, wobei die Eigenschaft der Trainingseinheit oder des Wettkampfs aus der Gruppe ausgewählt ist, bestehend aus einer speziellen Sportart, einer Zeitdauer, einer durch die Person gespielten Position, einem Ort, einer Umgebungstemperatur an dem Ort, einer Luftfeuchtigkeit an dem Ort, einer Windgeschwindigkeit an dem Ort und Kombinationen davon.

4. System zum Vorbeugen von Dehydratation bei sportlichem Training nach einem der vorstehenden Ansprüche, wobei die Benutzereingabe die Zeitdauer der Trainingseinheit oder des Wettkampfs, das Gewicht der Person, die Größe der Person, das Alter der Person, das Geschlecht der Person, die Umgebungstemperatur an dem Ort der Trainingseinheit oder des Wettkampfs und optional die Windgeschwindigkeit an dem Ort der Trainingseinheit oder des Wettkampfs umfasst, und wobei die Vielzahl von Anweisungen veranlasst, dass das Hydratationsberechnungsmodul den Wasserverlust in Schweiß der Person mindestens teilweise basierend auf den Daten bestimmt.

5. System zum Vorbeugen von Dehydratation bei sportlichem Training nach einem der vorstehenden Ansprüche, wobei die Benutzereingabe eine Zeitdauer der Trainingseinheit oder des Wettkampfs, die Ruheherzfrequenz der Person, das Alter der Person und optional die VO$_2$max der Person umfasst, und wobei die Vielzahl von Anweisungen veranlasst, dass das Hydratationsberechnungsmodul den Wasserverlust in Urin der Person mindestens teilweise basierend auf den Daten bestimmt.

**6.** System zum Vorbeugen von Dehydratation bei sportlichem Training nach einem der vorstehenden Ansprüche, wobei die Benutzereingabe eine Zeitdauer der Trainingseinheit oder des Wettkampfs, das Gewicht der Person, die Größe der Person, das Alter der Person, das Geschlecht der Person, die Umgebungstemperatur an dem Ort der Trainingseinheit oder des Wettkampfs und optional die Windgeschwindigkeit an dem Ort der Trainingseinheit oder des Wettkampfs umfassen, und wobei die Vielzahl von Anweisungen veranlasst, dass das Hydratationsberechnungsmodul den Wasserverlust durch Atmung der Person mindestens teilweise basierend auf den Daten bestimmt.

**7.** System zum Vorbeugen von Dehydratation bei sportlichem Training nach einem der vorstehenden Ansprüche, wobei die Benutzereingabe eine Zeitdauer der Trainingseinheit oder des Wettkampfs, das Gewicht der Person, die Größe der Person und die Umgebungstemperatur an dem Ort der Trainingseinheit oder des Wettkampfs umfasst, und wobei die Vielzahl von Anweisungen veranlasst, dass das Hydratationsberechnungsmodul den unmerklichen Wasserverlust mindestens teilweise basierend auf den Daten bestimmt.

**8.** System zum Vorbeugen von Dehydratation bei sportlichem Training nach den vorstehenden Ansprüchen, wobei die Vielzahl von Anweisungen veranlasst, dass das Hydratationsberechnungsmodul eine anfängliche Hauttemperatur der Person und eine endgültige Hauttemperatur der Person für die Trainingseinheit oder den Wettkampf bestimmt und den personalisierten Hydratationsplan mindestens teilweise basierend auf der anfänglichen und der endgültigen Hauttemperatur bestimmt.

**9.** System zum Vorbeugen von Dehydratation bei sportlichem Training nach den vorstehenden Ansprüchen, wobei die Benutzereingabe eine Zeitdauer der Trainingseinheit oder des Wettkampfs, das Gewicht der Person, das Alter der Person und das Geschlecht der Person umfasst, und wobei die Vielzahl von Anweisungen veranlasst, dass das Hydratationsberechnungsmodul die Menge an endogenem Wasser, die durch die Person ausgebildet wird, mindestens teilweise basierend auf der Benutzereingabe bestimmt.

**10.** System zum Vorbeugen von Dehydratation bei sportlichem Training nach einem der vorstehenden Ansprüche, wobei die Vielzahl von Anweisungen veranlasst, dass das Hydratationsberechnungsmodul die Benutzereingabe analysiert, um eine Magenentleerungsrate der Person zu bestimmen und um eine empfohlene Häufigkeit der Wasseraufnahme mindestens teilweise basierend auf der Magenentleerungsrate der Person zu bestimmen.

**11.** System zum Vorbeugen von Dehydratation bei sportlichem Training nach einem der vorstehenden Ansprüche, wobei die Vielzahl von Anweisungen veranlasst, dass das Hydratationsberechnungsmodul die Benutzereingabe analysiert, um einen Verlust von mindestens einem Mineralstoff, der aus der Gruppe ausgewählt ist, bestehend aus Natrium, Calcium und Magnesium, während der Trainingseinheit oder des Wettkampfs zu bestimmen und um eine empfohlene Aufnahmemenge des mindestens einen Mineralstoffs für die Person zu bestimmen.

**12.** System zum Vorbeugen von Dehydratation bei sportlichem Training nach einem der vorstehenden Ansprüche, wobei die Vielzahl von Anweisungen veranlasst, dass das Hydratationsberechnungsmodul die Benutzereingabe analysiert, um einen Glucoseverlust während der Trainingseinheit oder des Wettkampfs zu bestimmen und um eine empfohlene Glucoseaufnahmemenge für die Person zu bestimmen.

**13.** System zum Vorbeugen von Dehydratation bei sportlichem Training nach den vorstehenden Ansprüchen, wobei das Hydratationseingabemodul konfiguriert ist, um die Benutzereingabe zu empfangen, und das Hydratationsberechnungsmodul konfiguriert ist, um den personalisierten Hydratationsplan zu bestimmen, bevor der Benutzer an der Trainingseinheit oder dem Wettkampf teilnimmt.

**14.** System zum Vorbeugen von Dehydratation bei sportlichem Training nach einem der vorstehenden Ansprüche, wobei der personalisierte Hydratationsplan die sportliche Leistung der Person während der Trainingseinheit oder des Wettkampfs verbessert.

**15.** System zum Vorbeugen von Dehydratation bei sportlichem Training nach einem der vorstehenden Ansprüche, wobei die Benutzereingabe eine Identifizierung einer speziellen Sportart einschließt und die Vielzahl von Anweisungen veranlasst, dass das Hydratationsberechnungsmodul die Benutzereingabe für eine erste Sportart unter Verwendung mindestens einer Variablen oder Gleichung analysiert, die sich von der Analyse der Benutzereingabe für eine andere zweite Sportart unterscheidet, selbst wenn die Informationen ansonsten identisch sind.

**Revendications**

1. Système de prévention de la déshydratation lors d'un entraînement sportif permettant de diminuer ou de prévenir la déshydratation chez un individu lors d'une séance d'entraînement sportif ou d'un match, comprenant :

   un module d'entrée d'hydratation configuré pour recevoir au moins une entrée utilisateur représentant des informations choisies dans le groupe constitué d'une caractéristique de l'individu, d'une caractéristique de la séance d'entraînement ou du match, et d'une combinaison de celles-ci ;
   un module de calcul d'hydratation comprenant au moins un processeur et au moins un dispositif de mémoire qui stocke une pluralité d'instructions qui, lorsqu'elles sont exécutées par l'au moins un processeur, (a) analysent les informations fournies par l'entrée utilisateur pour déterminer une perte d'eau par l'individu au cours de la séance d'entraînement sportif ou du match, sur la base de la détermination de la perte d'eau totale en ajoutant une perte d'eau par la sueur, une perte d'eau dans l'urine, une perte d'eau respiratoire, une perte d'eau insensible, et en soustrayant l'eau endogène formée pendant la séance d'entraînement sportif ou le match, et (b) déterminent un plan d'hydratation personnalisé pour l'individu sur la base de l'entrée utilisateur ; et
   un module de sortie configuré pour amener un dispositif d'affichage à afficher des aspects du plan d'hydratation personnalisé, comportant au moins un élément choisi dans le groupe constitué : d'une quantité d'eau recommandée pour l'individu sur la base de la perte d'eau déterminée et d'une fréquence de consommation d'eau sur la base de la perte d'eau déterminée, et dans lequel au moins une partie de la quantité d'eau est identifiée pour être consommée avant et/ou pendant et/ou après la séance d'entraînement ou le match,
   dans lequel :

   - la perte d'eau par la sueur est de

   $$S = (60 * a * E_{req} * E_{max}^{-b} * t * Bsa)/\lambda f$$

   avec $E_{req}$ = refroidissement par évaporation nécessaire (W.°C$^{-1}$ m$^{-2}$), $E_{max}$ = capacité d'évaporation maximale (W.mmHg$^{-1}$.m$^{-2}$), $\lambda f$ = chaleur de vaporisation finale (J.g$^{-1}$), Bsa = surface corporelle (m$^2$) et t = durée de l'effort (min)
   - la perte d'eau dans l'urine est de

   $$U = a * 10^{-7} * HRr * (67 + 20,61 * ((VO_2 max * poids/1000)-2,0)) * t$$

   avec a = 2,16 pour les hommes et a = 2,36 pour les femmes, t = durée de l'effort (min), HRr = fréquence cardiaque au repos (bpm), $VO_2$ max = consommation maximale d'oxygène (mL.min$^{-1}$.kg$^{-1}$)
   - la perte d'eau respiratoire est de

   $$R = 0,0023 * M * (P_{sres}-P_a) * Bsa * 60 * t/\lambda$$

   avec M = chaleur métabolique (W.m$^2$), $P_{sres}$ = pression de vapeur d'eau saturée des voies respiratoires (mmHg), $P_a$ = pression de vapeur d'eau ambiante (mmHg), Bsa = surface corporelle (m$^2$), $\lambda$ = chaleur latente de vaporisation (J.g$^{-1}$), t = durée de l'effort (min)
   - la perte insensible d'eau est de

   $$I_n = (6,0 + 1,75 * (P_{Sp} - P_a)/7,5) * Bsa * t$$

   avec $P_{sp}$ = pression de vapeur d'eau de la peau (mmHg), $P_a$ = pression de vapeur d'eau ambiante (mmHg), Bsa = surface corporelle (m$^2$), t = durée de l'effort (min), et
   - l'eau endogène formée est de

   $$m_{met} = m_{met\varepsilon} + m_{metglyco}$$

   avec $m_{met\varepsilon}$ = (VO$_2$ * p * t * e * 10$^{-6}$) * (0,0144 + (0,1417 * QR) - Neo * (0,00000031 * t + 0,00000904)), et $m_{metglyco}$ = (VO$_2$ * p * t * e * 10$^{-6}$) * (-1,417 + (2,0012 * (0,99944)$^t$) - (0,000494 * t) - Neo * (0,000000975 * t + 0,0000313) + Hep * (0,00000591 * t + 0,000191)), avec VO$_2$ = consommation d'oxygène (mL.min$^{-1}$.kg$^{-1}$), p = poids (kg), t = durée de l'effort (min), e = équivalent énergétique en oxygène (kcal.L$^{-1}$), QR = quotient

respiratoire (sans unité), Neo = rapport de glucose fourni par la néoglucogenèse (%), Hep = rapport de glucose fourni par le glycogène hépatique (%).

2. Système de prévention de la déshydratation lors d'un entraînement sportif selon la revendication 1, dans lequel la caractéristique de l'individu est choisie dans le groupe constitué du poids, de la taille, du sexe, de la fréquence cardiaque au repos, de la fréquence cardiaque maximale, de la $VO_2$ max, de l'intensité de l'effort pour la séance d'entraînement ou le match, d'un type de vêtements portés pendant la séance d'entraînement ou le match, et des combinaisons de ceux-ci.

3. Système de prévention de la déshydratation lors d'un entraînement sportif selon la revendication 1 ou 2, dans lequel la caractéristique de la séance d'entraînement ou du match est choisie dans le groupe constitué d'un sport spécifique, d'une durée, d'une position jouée par l'individu, d'un lieu, d'une température ambiante sur le lieu, d'une humidité sur le lieu, d'une vitesse du vent sur le lieu, et des combinaisons de ceux-ci.

4. Système de prévention de la déshydratation lors d'un entraînement sportif selon l'une quelconque des revendications précédentes, dans lequel l'entrée utilisateur comprend la durée de la séance d'entraînement ou du match, le poids de l'individu, la taille de l'individu, l'âge de l'individu, le sexe de l'individu, la température ambiante sur le lieu de la séance d'entraînement ou du match, et éventuellement, la vitesse du vent sur le lieu de la séance d'entraînement ou du match, et dans lequel la pluralité d'instructions amène le module de calcul de l'hydratation à déterminer la perte d'eau dans la sueur par l'individu sur la base, au moins en partie, des données.

5. Système de prévention de la déshydratation lors d'un entraînement sportif selon l'une quelconque des revendications précédentes, dans lequel l'entrée utilisateur comprend la durée de la séance d'entraînement ou du match, la fréquence cardiaque au repos de l'individu, l'âge de l'individu et, éventuellement, la $VO_2$ max de l'individu, et dans lequel la pluralité d'instructions amène le module de calcul de l'hydratation à déterminer la perte d'eau dans l'urine par l'individu sur la base, au moins en partie, des données.

6. Système de prévention de la déshydratation lors d'un entraînement sportif selon l'une quelconque des revendications précédentes, dans lequel l'entrée utilisateur comprend une durée de la séance d'entraînement ou du match, le poids de l'individu, la taille de l'individu, l'âge de l'individu, le sexe de l'individu, la température ambiante sur le lieu de la séance d'entraînement ou du match, et éventuellement, la vitesse du vent sur le lieu de la séance d'entraînement ou du match, et dans lequel la pluralité d'instructions amène le module de calcul de l'hydratation à déterminer la perte d'eau respiratoire par l'individu sur la base, au moins en partie, des données.

7. Système de prévention de la déshydratation lors d'un entraînement sportif selon l'une quelconque des revendications précédentes, dans lequel l'entrée utilisateur comprend une durée de la séance d'entraînement ou du match, le poids de l'individu, la taille de l'individu et la température ambiante sur le lieu de la séance d'entraînement ou du match, et dans lequel la pluralité d'instructions amène le module de calcul de l'hydratation à déterminer la perte d'eau insensible sur la base, au moins en partie, des données.

8. Système de prévention de la déshydratation lors d'un entraînement sportif selon les revendications précédentes, dans lequel la pluralité d'instructions amène le module de calcul de l'hydratation à déterminer une température initiale de la peau de l'individu et une température finale de la peau de l'individu pour la séance d'entraînement ou le match et à déterminer le plan d'hydratation personnalisé, sur la base, au moins en partie, des températures initiale et finale de la peau.

9. Système de prévention de la déshydratation lors d'un entraînement sportif selon les revendications précédentes, dans lequel l'entrée utilisateur comprend une durée de la séance d'entraînement ou du match, le poids de l'individu, l'âge de l'individu et le sexe de l'individu, et dans lequel la pluralité d'instructions amène le module de calcul de l'hydratation à déterminer la quantité d'eau endogène formée par l'individu sur la base, au moins en partie, de l'entrée utilisateur.

10. Système de prévention de la déshydratation lors d'un entraînement sportif selon l'une quelconque des revendications précédentes, dans lequel la pluralité d'instructions amène le module de calcul de l'hydratation à analyser l'entrée utilisateur à déterminer la vitesse de vidange gastrique de l'individu et à déterminer une fréquence recommandée de consommation d'eau sur la base, au moins en partie, de la vitesse de vidange gastrique de l'individu.

11. Système de prévention de la déshydratation lors d'un entraînement sportif selon l'une quelconque des revendications précédentes, dans lequel la pluralité d'instructions amène le module de calcul de l'hydratation à analyser l'entrée utilisateur pour déterminer une perte, au cours de la séance d'entraînement ou du match, d'au moins un minéral choisi dans le groupe constitué de sodium, de calcium et de magnésium, et à déterminer une quantité recommandée de consommation d'au moins un minéral pour l'individu.

12. Système de prévention de la déshydratation lors d'un entraînement sportif selon l'une quelconque des revendications précédentes, dans lequel la pluralité d'instructions amène le module de calcul de l'hydratation à analyser l'entrée utilisateur afin de déterminer une perte de glucose au cours de la séance d'entraînement ou du match et de déterminer une quantité recommandée de consommation de glucose pour l'individu.

13. Système de prévention de la déshydratation lors d'un entraînement sportif selon les revendications précédentes, dans lequel le module d'entrée d'hydratation est configuré pour recevoir l'entrée utilisateur, et le module de calcul d'hydratation est configuré pour déterminer le plan d'hydratation personnalisé, avant que l'utilisateur ne participe à la séance d'entraînement ou au match.

14. Système de prévention de la déshydratation lors d'un entraînement sportif selon l'une quelconque des revendications précédentes, dans lequel le plan d'hydratation personnalisé améliore les performances sportives de l'individu pendant la séance d'entraînement ou le match.

15. Système de prévention de la déshydratation lors d'un entraînement sportif selon l'une quelconque des revendications précédentes, dans lequel l'entrée utilisateur comprend une identification d'un sport spécifique, et la pluralité d'instructions amène le module de calcul de l'hydratation à analyser l'entrée utilisateur pour un premier sport à l'aide d'au moins une variable ou d'une équation différente de l'analyse de l'entrée utilisateur pour un second sport différent, même si les informations sont par ailleurs identiques.

# FIG. 1

# FIG. 2

Body weight loss (% of initial body weight)

# FIG. 3

Basket France Team junior
Individual water losses during training

FIG. 4

## French pro soccer team
## Individual water losses during training

# FIG. 5

French pro cycling team
Individual water losses during training

**FIG. 6**

French elite woman tennis team
Individual water losses during training

## FIG. 7

Elite handball teams : global water losses during training & match with various conditions

# FIG. 8

**Water losses at ice hockey players of different age classes**

## FIG. 9

Comparison of water losses : women versus men in tennis training

# FIG. 10

# FIG. 11

○ If VO2max is known : data

○ If VO2max not known :

   ◉ If Cooper test distance is known and introduced as data ($d_c$, km) :

$$\boxed{\text{VO2max} = 22.351 * d_c - 11.288}$$

   ◉ If calculation is requested :

$$\text{Data (A = age)}$$

$$\downarrow$$

$$\boxed{\begin{array}{c} 185.32 * A^{(-0.426)} \\ \| \quad \text{If sex = 1} \\ \text{VO2max} \\ \text{If sex = 2} \quad \| \\ 53.68 * (0.989)^{A} \end{array}}$$

# FIG. 12

What is heart rate at rest ($Fc_r$) ?
If known, $Fc_r$ = data
If not, $Fc_r$ = 65

calculations in Fig. 11

$$2.16 * 10^{-7} * Fc_r * [67 + 20.61 * [(VO_{2max} * p / 1000) - 2.0]] * t$$

|| If sex = 1

$$m_u$$

If sex = 2   ||

$$2.36 * 10^{-7} * Fc_r * [67 + 20.61 * [(VO_{2max} * p / 1000) - 2.0]] * t$$

Data

Module

# FIG. 13

**FIG. 14**

Module

Module

$$5.167 * (0.9998856^{t})$$

Data

$$m_{met\varepsilon} = (VO2 * p * t * e / 10^{6}) *$$
$$[0.0144 + (0.1417 * QR) - Neo * (0.00000031 * t + 0.00000904)]$$

Module

$$m_{met} = (m_{met\varepsilon}) / 1000$$

FIG. 15

$P_{met}$ = Metabolic weight loss (kg)

Module

Data

Calculations from FIG. 10

VO2*p*t*[-1.429-(0.2567*e)+QR*(1.977+0.3624*e)
-Neo*e*(0.000000604*t+0.00001742)] / 10⁶

Module

**FIG. 16**

① **If t ≤ 180**

mGlu = VO2*p*t*e*[-0.538+(0.8018*QR)- Néo*(0.000002616*t+0.00007556)]/1000

Data

$5.167 * (0.9998856)^t$

Fig. 10

② **If t > 180**

mGlu = VO2*p*180*e*[-0.538+(0.78496*(0.99944)^t)- 0.01858)]/1000

**FIG. 17**

$$Glu_{LEC} = 0.9 * m_m * 0.22$$

$$Gly_f = (Glu_{LEC} + ED) / (0.22 * m_m)$$

$$ED = Glu_{circU} * 10^{-2} * (Néo + Hep - 100)$$

$$Glu_{circU} = VO_2 * p * t * e * 10^{-3} * [0.0002031 * t + 0.00654]$$

**FIG. 18**

**FIG. 19**

$$m_b = m_{sf} + m_u + m_{res} - m_{met} - V_e$$

$$Pp1 = (m_b * 100) / (p - p_{met})$$

**FIG. 20**

$> 0.2$ —— [meau - (Aeff * n)] —— $\leq 0.2$

Afin = [meau - (Aeff * n)]

Afin = «Drink according to your thirst»

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6138079 A

**Non-patent literature cited in the description**

- **CASA DJ et al.** *J Athl Train*, September 2015, vol. 50 (9), 986-1000
- **CORIS EE et al.** *Sports Med.*, 2004, vol. 34 (1), 9-16
- **BARDIS CN**. *Medicine and Science in Sports and Exercise*, September 2013, vol. 45 (9), 1782-1789
- **POORTMANS JR**. *Sports Medicine*, 1984, vol. 1, 125-153

- The stress of hot environments. **KERSLAKE D. MCK.** Monographs of the Physiological Society. Cambridge, University Press
- **FERRUS L. et al.** *Respiration Physiology*, 1980, vol. 39, 367-381
- **TOKURA et al.** *Int. J. Biometeor.*, 1978, vol. 22 (4), 271-278
- **MATHIAS et al.** *J. Invest. Dermatol.*, 1981, vol. 77, 219-220
- **WILSON et al.** *Br. J. Dermatol.*, 1988, vol. 119, 647-652